# EUROPEAN PATENT APPLICATION

(11) **EP 4 817 633 A1**
(43) Date of publication of application: **07.10.2026**
(21) Application number: 24897668.0
(22) Date of filing: 28.11.2024
(51) Int. Cl.: A61K 45/00, A61K 33/44, A61P 9/12, A61P 11/00, A61P 39/02, G01N 33/68

(54) **PROPHYLACTIC OR THERAPEUTIC AGENT FOR PULMONARY HYPERTENSION, AND METHOD FOR EXAMINING PRESENCE OR ABSENCE OF PULMONARY HYPERTENSION OR SEVERITY OF PULMONARY HYPERTENSION**

(30) Priority: 29.11.2023 JP 2023202282
(71) Applicant: National Cerebral and Cardiovascular Center, Suita-shi, Osaka 564-8565 (JP)
(72) Inventor: NAKAOKA, Yoshikazu, Kishibe-Shimmachi, Suita-shi, Osaka 564-8565 (JP); OKAZAWA, Makoto, Kishibe-Shimmachi, Suita-shi, Osaka 564-8565 (JP); ASANO, Ryotaro, Kishibe-Shimmachi, Suita-shi, Osaka 564-8565 (JP)
(74) Representative: Hoffmann Eitle
(86) International application number: PCT/JP2024/042236
(87) International publication number: WO 2025/115984

(57) **Abstract**

An object of the present disclosure is to provide a prophylactic or therapeutic agent for pulmonary hypertension. A prophylactic or therapeutic agent for pulmonary hypertension according to the present disclosure contains a substance that adsorbs or binds to indole compounds that are tryptophan metabolites.

## Description

### Technical Field

The present disclosure relates to a prophylactic or therapeutic agent for pulmonary hypertension (PH). The present disclosure also relates to a method for examining the presence or absence of PH or the severity of PH, and an examination kit used in the examination method.

### Background Art

Pulmonary hypertension is a group of progressive diseases having a poor prognosis that cause functional disorders of the heart and the lungs due to an increase in blood pressure in a pulmonary artery, and is classified, according to the Nice classification, into Group 1: pulmonary arterial hypertension (PAH); Group 1': pulmonary veno-occlusive disease (PVOD) and/or pulmonary capillary hemangiomatosis (PCH); Group 1'': persistent pulmonary hypertension of a newborn; Group 2: pulmonary hypertension associated with a left heart disease; Group 3: pulmonary hypertension associated with a lung disease and/or hypoxemia; Group 4: chronic thromboembolic pulmonary hypertension (CTEPH); and Group 5: pulmonary hypertension associated with a multifactorial mechanism of unknown details.

Among PH, PAH is a disease having, as a main site of inflammation, a peripheral pulmonary artery (an arteriole or a small artery), accompanied by pathological changes such as muscularization of a pulmonary artery, neointimal proliferation, and plexiform lesions (NPL 1 and NPL 2). The etiology of PAH is multifactorial, and is presumably caused by a combination of various factors such as a genetic background, an epigenetic modification factor, and a pre-existing disease or an environmental factor (NPL 3). Among such factors, inflammation and an autoimmune disease are considered to greatly contribute to the etiology of PAH (NPL 4 and NPL 5).

In recent years, although the overall prognosis of PH has been improving by vasodilators targeting relaxation of a smooth muscle, the prognosis of a severe patient or a drug-resistant patient is still considerably poor. The onset and aggravation of PH are considered to be importantly influenced by a second hit such as inflammation or a drug in addition to a genetic predisposition, but many points of the details remain unclear.

The present inventors have reported that an aryl hydrocarbon receptor (AHR), which is a regulator of an inflammatory signal and is known as a receptor for a pollutant in an environment, plays a key role in pathogenesis of PAH (NPL 6). In the present report, it was found that, in a PAH patient, a serum AHR activity was high, the activity was higher as the severity was greater, and a group having a high activity was associated with a poor prognosis (Fig. 1). Furthermore, it has been reported that FICZ, an endogenous AHR ligand, induces severe pulmonary hypertension accompanied by intimal lesions in rats when combined with hypoxic stimulation (Fig. 2). Further, it has been reported that in the Su/Hx model, which is a severe PH model, knockout of AHR almost completely suppresses the pathogenesis of PH, and that AHR plays a key role in the aggravation of PH (Fig. 3).

### Citation List

### Non Patent Literature

NPL 1: Humbert M, Sitbon O, Simonneau G. Treatment of pulmonary arterial hypertension. The New England journal of medicine 2004;351:1425-36.
NPL 2: Rabinovitch M. Molecular pathogenesis of pulmonary arterial hypertension. The Journal of clinical investigation 2012;122:4306-13.
NPL 3: Humbert M, Guignabert C, Bonnet S, et al. Pathology and pathobiology of pulmonary hypertension: state of the art and research perspectives. The European respiratory journal 2019;53.
NPL 4: Hashimoto-Kataoka T, Hosen N, Sonobe T, et al. Interleukin-6/interleukin-21 signaling axis is critical in the pathogenesis of pulmonary arterial hypertension. Proceedings of the National Academy of Sciences of the United States of America 2015;112:E2677-86.
NPL 5: Schermuly RT, Ghofrani HA, Wilkins MR, Grimminger F. Mechanisms of disease: pulmonary arterial hypertension. Nature reviews Cardiology 2011;8:443-55.
NPL 6: Masaki T. Okazawa M et al., Aryl hydrocarbon receptor is essential for the pathogenesis of pulmonary arterial hypertension. PNAS 2021; 118, e2023899118

### Summary of Invention

### Technical Problem

When a ligand of an AHR, which plays an important role in pathogenesis of PH, can be removed, the removal may be effective for prevention of onset of PH, treatment of PH, and prevention of aggravation of PH. However, conventionally, an AHR ligand involved in pathogenesis of PH has not been identified.

Therefore, in one embodiment of the present disclosure, an object is to identify an AHR ligand involved in pathogenesis of PH and to provide a prophylactic or therapeutic agent for PH capable of removing the AHR ligand.

Further, in another embodiment of the present disclosure, an object is to elucidate a mechanism of pathogenesis of PH caused by an AHR ligand and to provide a novel prophylactic or therapeutic agent for PH based on the mechanism.

Further, in still another embodiment of the present disclosure, an object is to find a biomarker for PH and to provide a method for examining the presence or absence of PH or the severity of PH.

### Solution to Problem

It has been reported that a change in a metabolite due to dysbiosis of a gut microbiota is associated with various diseases. Therefore, the present inventors analyzed a gut microbiota of a PH patient. As a result, it was found that, in a PH patient, dysbiosis of a gut microbiota characterized by ectopic colonization of oral bacteria in the intestine and an increase in Ruminococcus gnavus (R. gnavus) occurs. Furthermore, it was found that this dysbiosis of a gut microbiota resulted in high concentrations of tryptophan in an intestinal tract of a PH patient, which was accompanied by an enhanced production of tryptophan metabolites (indole compounds) such as tryptamine. Furthermore, it was found that tryptophan metabolites function as AHR ligands and promote the pathogenesis of PH. Furthermore, it was found that, in the intestinal tract, tryptophan metabolites (AHR ligands) such as tryptamine inhibit the absorption of tryptophan by decreasing B0AT1 function through an AHR-dependent reduction in the expression level of angiotensin-converting enzyme 2 (ACE2), thereby further promoting high concentrations of tryptophan in the intestinal tract. Further, it was found that PH can be prevented or treated by administering a substance that adsorbs or binds to a tryptophan metabolite to remove the tryptophan metabolite from the intestinal tract. The present disclosure has been completed through further studies based on these findings.

That is, in one embodiment of the present disclosure, the present disclosure relates to a technique for prevention or treatment of PH having a mechanism of removing tryptophan metabolites in the intestinal tract, and provides the inventions described below.
Item 1-1. A prophylactic or therapeutic agent for PH, containing a substance capable of removing indole compounds that are tryptophan metabolites from an intestinal tract.
Item 1-2. The prophylactic or therapeutic agent for PH according to item 1-1, in which the substance is a substance that adsorbs or binds to indole compounds that are tryptophan metabolites.
Item 1-3. The prophylactic or therapeutic agent for PH according to item 1-1 or 1-2, in which the indole compound is at least one selected from the group consisting of tryptamine, indole-3-aldehyde, indole-3-pyruvic acid, indole-3-ethanol, indole-3-acetic acid, indole-3-propionic acid, indole-3-acrylic acid, and indole-3-lactic acid.
Item 1-4. The prophylactic or therapeutic agent for PH according to item 1-1 or 1-2, in which the substance is a porous inorganic adsorbent.
Item 1-5. The prophylactic or therapeutic agent for PH according to any one of items 1-1 to 1-4, in which the substance is a carbonaceous adsorbent.
Item 1-6. A method for treating PH, including a step of removing indole compounds that are tryptophan metabolites from an intestinal tract of a PH patient.
Item 1-7. The method for treating PH according to item 1-6, in which indole compounds that are tryptophan metabolites are removed from an intestinal tract of a PH patient by administering a therapeutically effective amount of a substance that adsorbs or binds to the indole compounds, to the PH patient.
Item 1-8. Use of a substance capable of removing indole compounds that are tryptophan metabolites from an intestinal tract for the manufacture of a prophylactic or therapeutic agent for PH.
Item 1-9. Use according to item 1-8, in which the substance is a substance that adsorbs or binds to indole compounds that are tryptophan metabolites.
Item 1-10. A substance capable of removing indole compounds that are tryptophan metabolites from an intestinal tract, for use in a treatment of PH.
Item 1-11. A substance that adsorbs or binds to indole compounds that are tryptophan metabolites, for use in a treatment of PH.

Further, another embodiment of the present disclosure relates to a technique for examining the presence or absence of PH or the severity of PH using the tryptophan concentrations in feces, intestinal contents, or blood samples as an indicator, and provides the following invention.
Item 2-1. A method for examining the presence or absence of PH or the severity of PH, including a step of measuring tryptophan concentrations in feces, intestinal contents, or blood samples collected from a subject.
Item 2-2. An examination kit for the presence or absence of PH or the severity of PH, including a reagent for measuring tryptophan.
Further, another embodiment of the present disclosure relates to a technique for examining the presence or absence of PH or the severity of PH using the concentration of tryptophan metabolites in feces, intestinal contents, or blood samples as an indicator, and provides the following invention.
Item 3-1. A method for examining the presence or absence of PH or the severity of PH, including a step of measuring concentrations of indole compounds that are metabolites of tryptophan in feces, intestinal contents, or blood samples collected from a subject.
Item 3-2. The method according to item 3-1, in which the indole compound is at least one selected from the group consisting of tryptamine, indole-3-aldehyde, indole-3-pyruvic acid, indole-3-ethanol, indole-3-acetic acid, indole-3-propionic acid, indole-3-acrylic acid, and indole-3-lactic acid.
Item 3-3. An examination kit for the presence or absence of PH or the severity of PH, including a reagent for measuring indole compounds that are tryptophan metabolites.

In addition, another embodiment of the present disclosure relates to a technique for examining the presence or absence of PH or the severity of PH using the AHR activity in feces or intestinal contents as an indicator, and provides the following invention.
Item 4-1. A method for examining the presence or absence of PH or the severity of PH, including a step of measuring the AHR activity in feces or intestinal contents collected from a subject.
Item 4-2. An examination kit for the presence or absence of PH or the severity of PH, including a reagent for measuring the AHR activity in feces or intestinal contents.

Further, another embodiment of the present disclosure relates to a technique for examining the presence or absence of PH or the severity of PH using the ACE2 concentrations in feces, intestinal contents, or blood samples as an indicator, and provides the following invention.
Item 5-1. A method for examining the presence or absence of PH or the severity of PH, including a step of measuring the ACE2 concentrations in feces, intestinal contents, or blood samples collected from a subject.
Item 5-2. An examination kit for the presence or absence of PH or the severity of PH, including a reagent for measuring ACE2.

In addition, another embodiment of the present disclosure relates to a technique for preventing or treating PH by the mechanism of an increase in ACE2 expression level in the intestinal tract, and provides the following inventions.
Item 6-1. A prophylactic or therapeutic agent for PH, containing a substance that increases the expression level of ACE2.
Item 6-2. A method for treating PH, including a step of administering a therapeutically effective amount of a substance that increases the expression level of ACE2 to a PH patient.
Item 6-3. Use of a substance that increases the expression level of ACE2 for the manufacture of a prophylactic or therapeutic agent for PH.
Item 6-4. A substance that increases the expression level of ACE2 used for treatment of PH.

### Advantageous Effects of Invention

According to an embodiment of the present disclosure, PH can be prevented or treated by removing the indole compounds that are tryptophan metabolites from the intestinal tract. In addition, according to another embodiment of the present disclosure, it is possible to prevent or treat PH by increasing the ACE2 expression level in the intestinal tract.

In addition, according to another embodiment of the present disclosure, the presence or absence of PH or the severity of PH can be examined using the tryptophan concentrations, the tryptophan metabolites, or the ACE2 concentrations in feces, intestinal contents, or blood samples as an indicator. Further, according to still another embodiment of the present disclosure, the presence or absence of PH or the severity of PH can be examined using the AHR activity in feces or intestinal contents as an indicator.

### Brief Description of Drawings

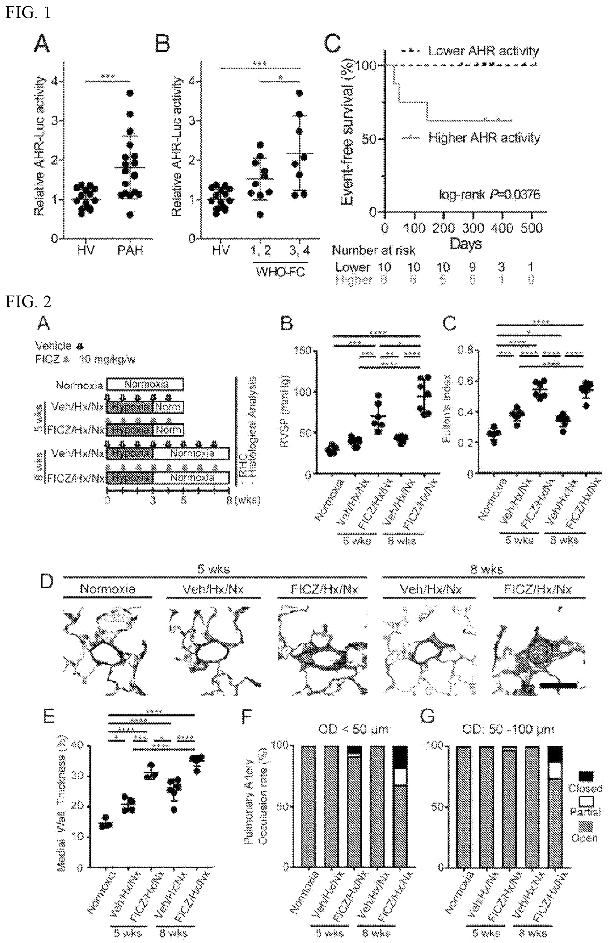

[Fig. 1] Fig. 1 is a diagram showing that AhR ligand activity in serum is increased in PAH patients, and that the results reflect the severity of PAH (NPL 6). A shows AHR-Luc activity in serum of healthy volunteers (HV) and PAH patients (HV: n = 16, PAH: n = 18), as determined by an AHR luciferase reporter assay; B shows a distribution of AHR-Luc (luciferase) activity stratified by WHO Functional Classification for pulmonary hypertension (HV: n = 16; WHO-FC 1, 2: n = 10; WHO-FC 3, 4: n = 8); and C shows a Kaplan-Meier analysis of event-free survival of patients with low AHR-Luc activity (n = 10) and patients with high AHR-Luc activity (n = 8) (P = 0.0376, two-sided log-rank test). In C, patients with low AHR-Luc activity and patients with high AHR-Luc activity were stratified using the mean value of AHR-Luc activity as a cutoff value. In C, clinical events were defined as death, lung transplantation, and hospitalization for treatment of right heart failure. The statistical values are mean ± standard deviation: ***P < 0.001, *P < 0.05.
[Fig. 2] Fig. 2 is a diagram showing results indicating that FICZ, an endogenous AHR ligand, in combination with hypoxic stimulation, induces severe pulmonary hypertension accompanied by intimal lesions in rats (NPL 6). A shows an experimental procedure for determining whether FICZ administration induces PH in rats in combination with hypoxic stimulation (FICZ/Hx/Nx model); B shows right ventricular systolic pressure (RVSP) in FICZ/Hx/Nx rats and Veh/Hx/Nx rats (Normoxia: n = 5, Veh/Hx/Nx 5 wks: n = 7, FICZ/Hx/Nx 5 wks: n = 6, Veh/Hx/Nx 8 wks: n = 6, FICZ/Hx/Nx 8 wks: n = 7); C shows a Fulton's index of the FICZ/Hx/Nx rats and the Veh/Hx/Nx rats; D shows representative vascular remodeling images of pulmonary distal small arteries in lung tissue sections of the rats of B and C stained with Elastica van Gieson (EVG); E shows a medial thickness index of the FICZ/Hx/Nx rats and the Veh/Hx/Nx rats (Normoxia: n = 3, Veh/Hx/Nx 5 wks: n = 4, FICZ/Hx/Nx 5 wks: n = 3, Veh/Hx/Nx 8 wks: n = 6, FICZ/Hx/Nx 8 wks: n = 6); F shows results obtained by grading pulmonary arterial occlusive lesions in vessels having a pulmonary arterial outer diameter (OD: < 50 µm) in the FICZ/Hx/Nx rats and the Veh/Hx/Nx rats as open (no intimal lesion), partial (< 50% occlusion), or closed (≥ 50% occlusion) (Normoxia: n = 3, Veh/Hx/Nx 5 wks: n = 4, FICZ/Hx/Nx 5 wks: n = 3, Veh/Hx/Nx 8 wks: n = 6, FICZ/Hx/Nx 8 wks: n = 6); and G shows results obtained by grading pulmonary arterial occlusive lesions in vessels having a pulmonary arterial outer diameter (OD: 50 to 100 µm) in the FICZ/Hx/Nx rats and the Veh/Hx/Nx rats as open, partial, or closed in the same manner as described above. In the drawing, the abbreviation "FICZ" indicates a FICZ administration group, "Veh" indicates a solvent administration group, "Hx" indicates a hypoxic load, and "Nx" indicates a normoxic condition. In A, the abbreviation "RHC" refers to right heart catheterization. The statistical values are mean ± standard deviation: ****P < 0.0001, ***P < 0.001, **P < 0.01, *P < 0.05.

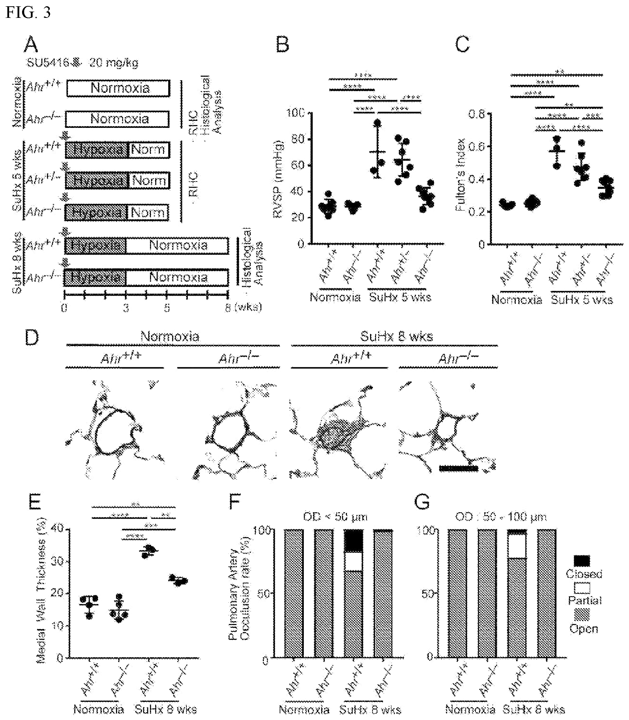

[Fig. 3] Fig. 3 is a diagram showing that AHR knockout rats were resistant to pulmonary hypertension in a SuHx rat model (NPL 6). A shows an experimental procedure for examining a role of an AHR signaling pathway in a SuHx rat model; B shows right ventricular systolic pressure (RVSP) at 5 weeks in SuHx rats (Ahr-/-, Ahr+/+) and rats maintained under normoxic conditions (Normoxia; Ahr-/-, Ahr+/+) (Normoxia Ahr+/+: n = 7, Normoxia Ahr-/-: n = 7, SuHx 5 wks Ahr+/+: n = 3, SuHx 5 wks Ahr+/-: n = 7, SuHx 5 wks Ahr-/-: n = 8); C shows a Fulton's index at 5 weeks in the SuHx rats (Ahr-/-, Ahr+/+) and the rats maintained under normoxic conditions (Normoxia; Ahr-/-, Ahr+/+); D shows representative vascular remodeling images of pulmonary distal small arteries in lung tissue sections at 8 weeks of the SuHx rats (Ahr-/-, Ahr+/+) and the rats maintained under normoxic conditions (Normoxia; Ahr-/-, Ahr+/+) stained with EVG; E shows a medial thickness index at 8 weeks in the SuHx rats (Ahr-/-, Ahr+/+) and the rats maintained under normoxic conditions (Normoxia; Ahr-/-, Ahr+/+) (Normoxia Ahr+/+: n = 4, Normoxia Ahr-/-: n = 5, SuHx 8 wks Ahr+/+: n = 3, SuHx 8 wks Ahr-/-: n = 3); F shows results obtained by grading pulmonary arterial occlusive lesions in vessels having a pulmonary arterial outer diameter (OD: < 50 µm) at 8 weeks in the SuHx rats (Ahr-/-, Ahr+/+) and the rats maintained under normoxic conditions (Normoxia; Ahr-/-, Ahr+/+) as open (no intimal lesion), partial (< 50% occlusion), or closed (≥ 50% occlusion); and G shows results obtained by grading pulmonary arterial occlusive lesions in vessels having a pulmonary arterial outer diameter (OD: 50 to 100 µm) at 8 weeks in the SuHx rats (Ahr-/-, Ahr+/+) and the rats maintained under normoxic conditions (Normoxia; Ahr-/-, Ahr+/+) as open, partial, or closed in the same manner as described above. In the drawing, the abbreviation "Su" indicates a SU5416 administration group, "Hx" indicates a hypoxic load, "Nx" indicates a normoxic condition, "Ahr-/-" indicates that AHR was knocked out, "Ahr+/+" indicates that AHR was not knocked out, and "RHC" indicates a right heart catheterization. The statistical values are mean ± standard deviation: ****P < 0.0001, ***P < 0.001, **P < 0.01.

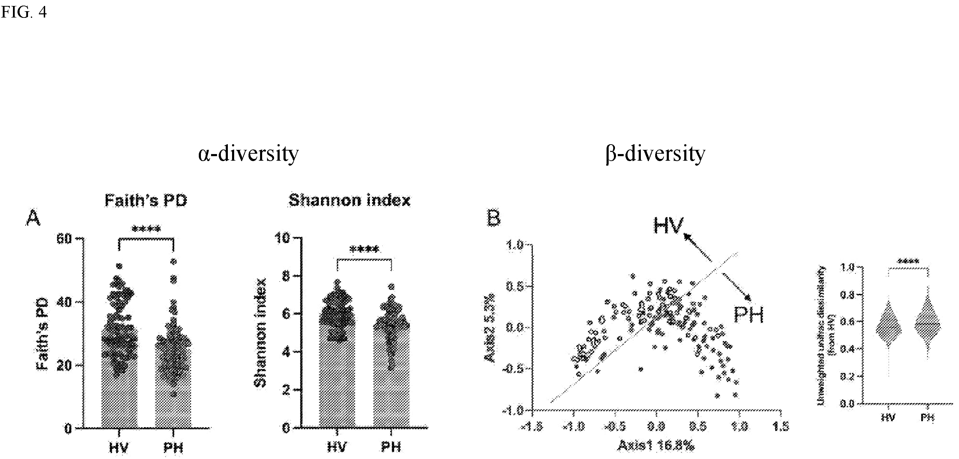

[Fig. 4] Fig. 4 is a diagram showing dysbiosis of a gut microbiota in a PH patient. A is a diagram showing α-diversity with respect to Faith PD and Shannon index for the gut microbiota of PH patients (PH, 89 cases) and healthy volunteers (HV, 82 cases). B is a diagram showing β-diversity (unweighted unifrac PCoA) of a gut microbiota of PH patients and healthy volunteers. ****P < 0.0001, *P < 0.05.

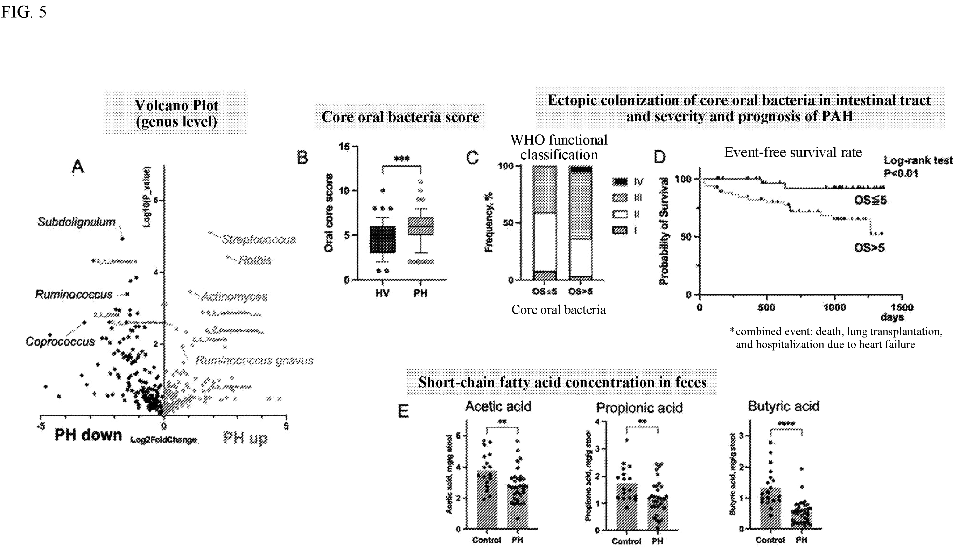

[Fig. 5] Fig. 5 is a diagram showing dysbiosis of a gut microbiota in a PH patient. A indicates a volcano plot comparing bacteria (genus level) constituting a gut microbiota between PH patients and healthy volunteers (HV). B shows an oral resident bacteria score in the intestine of PH patients and healthy volunteers. C shows results obtained by dividing PH patients into those having an oral resident bacteria score (OS) of 5 or more and those having an oral resident bacteria score of less than 5, and comparing proportions of PH functional classes I to IV of WHO. D shows results obtained by performing a Kaplan-Meier analysis of event-free survival (P < 0.01, two-sided log-rank test) by dividing PH patients into those having an oral resident bacteria score (OS) of 5 or more and those having an oral resident bacteria score of 5 or less. E shows respective concentrations of acetic acid, propionic acid, and butyric acid in feces of PH patients and healthy volunteers. In D, clinical events were defined as death, lung transplantation, and hospitalization for treatment due to right heart failure. The statistical values are mean ± standard deviation: ****P < 0.0001, ***P < 0.001, **P < 0.01, *P < 0.05.

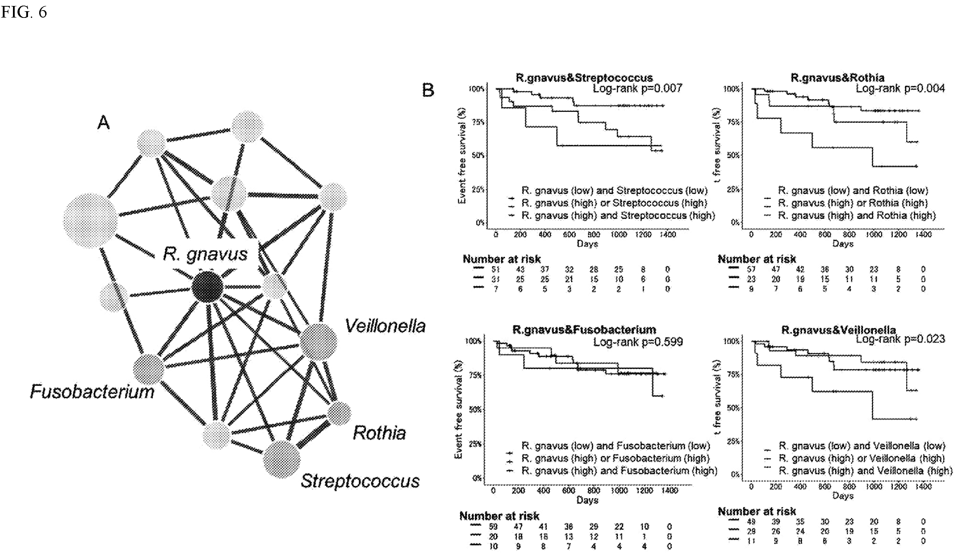

[Fig. 6] Fig. 6 is a diagram showing that in PH patients, Ruminococcus gnavus coexists by forming networks with multiple oral bacteria in the intestinal tract, which is associated with poor prognosis. A shows a network diagram with bacteria correlating with Ruminococcus gnavus, generated using the results of composition analysis of a gut microbiota of PH patients. B is a diagram showing results obtained by performing a Kaplan-Meier analysis of event-free survival by classifying PH patients according to the abundance of Ruminococcus gnavus and Streptococcus, Rothia, Fusobacterium, or Veillonella in the intestine. In B, clinical events were defined as death, lung transplantation, and hospitalization for treatment due to right heart failure.

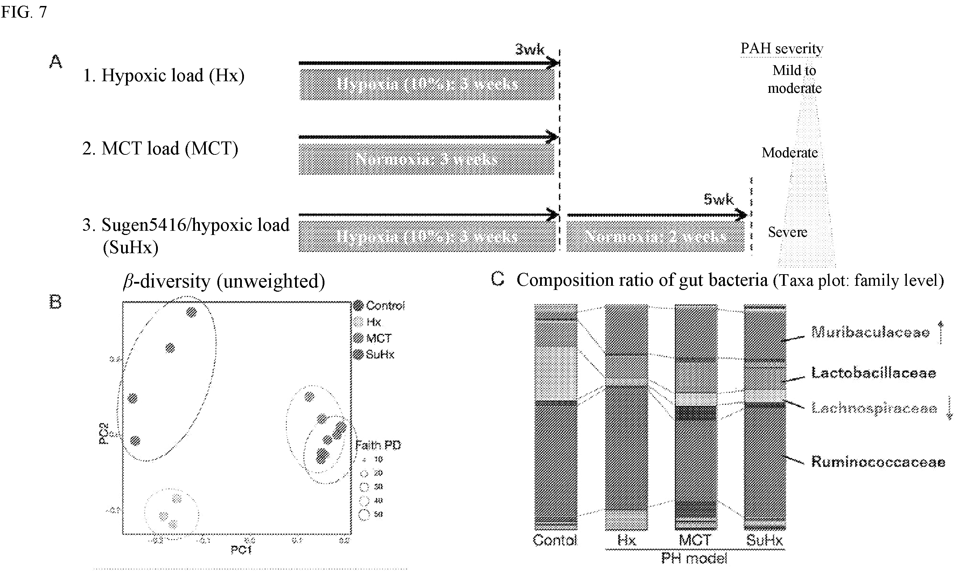

[Fig. 7] Fig. 7 is a diagram showing dysbiosis of a gut microbiota in pulmonary hypertension model rats (WO 2021/167088 A). A is a diagram showing procedures for preparing pulmonary hypertension model rats, in which an upper stage is for a hypoxic load, a middle stage is for monocrotaline load, and a lower stage is for Sugen5416/hypoxia/normoxia load (SuHx). B is a diagram showing β-diversity of a gut microbiota of each model rat. C is a diagram showing composition ratios of gut bacteria at a family level in a gut microbiota of each model rat as a bar plot.

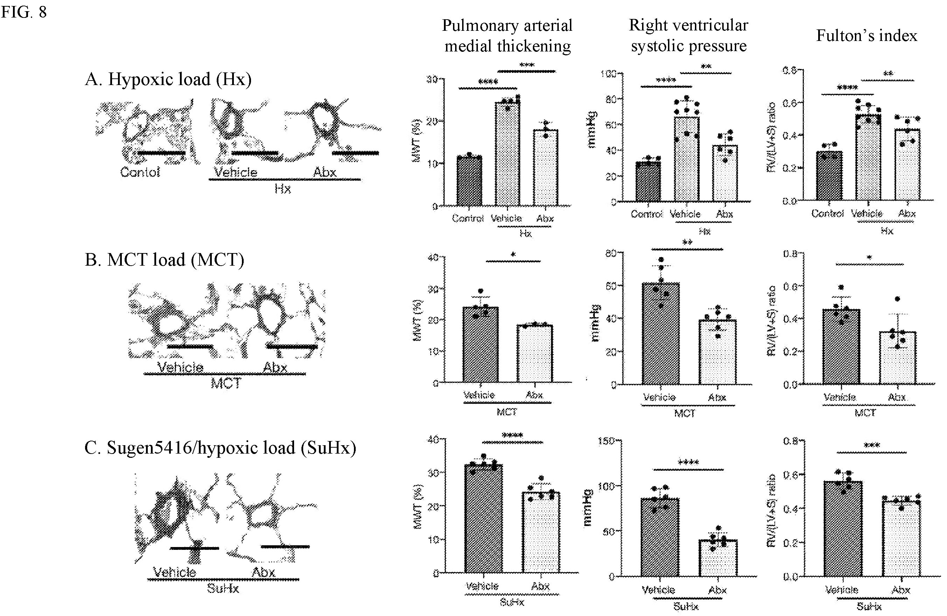

[Fig. 8] Fig. 8 is a diagram showing that PH pathology is improved by administering an antibiotic to the pulmonary hypertension model rats (WO 2021/167088 A). A shows results obtained by administering antibiotic cocktail water (Abx) or water (vehicle) by free drinking to hypoxia-loaded (Hx) model rats, and administering water by free drinking to SD rats without stimulation (Control), and evaluating PH pathology (pulmonary arterial medial thickening, right ventricular systolic pressure, and Fulton's index). B shows results obtained by administering antibiotic cocktail water (Abx) or water (vehicle) by free drinking to MCT model rats and evaluating PH pathology (pulmonary arterial medial thickening, right ventricular systolic pressure, and Fulton's index). C shows results obtained by administering antibiotic cocktail water (Abx) or water (vehicle) by free drinking to SuHx model rats and evaluating PH pathology (pulmonary arterial medial thickening, right ventricular systolic pressure, and Fulton's index). The statistical values are mean ± standard deviation: ****P < 0.0001, ***P < 0.001, **P < 0.01, *P < 0.05.

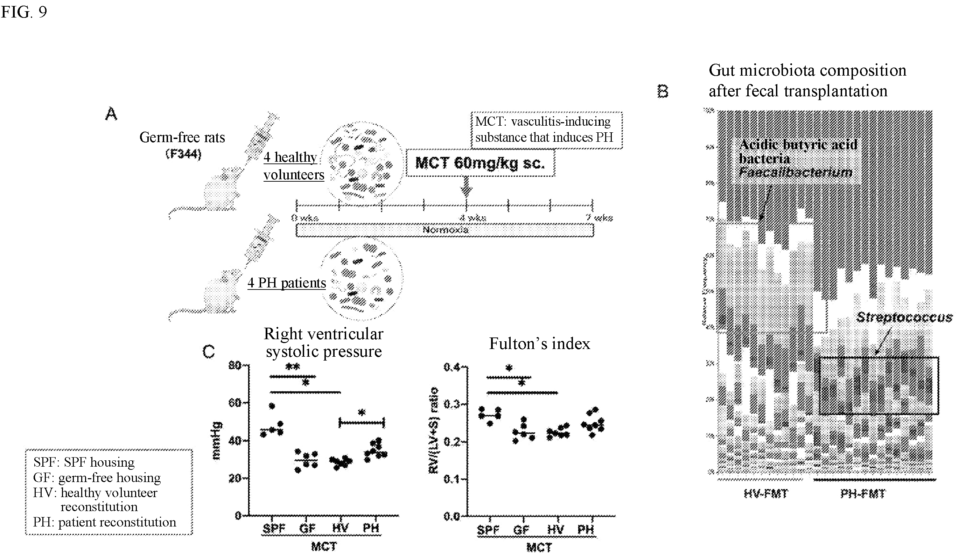

[Fig. 9] Fig. 9 is a diagram showing changes in a pulmonary hypertension pathology in PH model gnotobiotic rats. A is a diagram showing a method for preparing PH model gnotobiotic rats. B shows results obtained by analyzing a composition of a gut microbiota of PH patient feces-transplanted F344 rats and healthy volunteer feces-transplanted F344 rats. C shows results obtained by measuring right ventricular systolic pressure and Fulton's index of PH patient feces-transplanted F344 rats (MCT-PH), healthy volunteer feces-transplanted F344 rats (MCT-HV), germ-free environment-housed F344 rats (MCT-GF), and SPF environment-housed F344 rats (MCT-SPF). The statistical values are mean ± standard deviation. **P < 0.01, *P < 0.05.

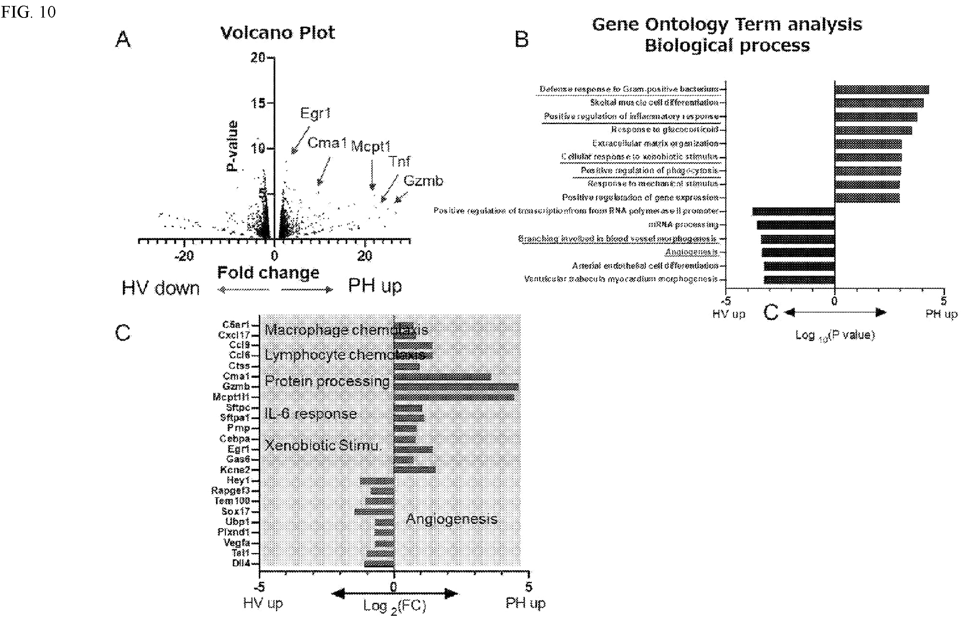

[Fig. 10] Fig. 10 is a diagram showing RNA-seq analysis results of lungs of PH patient feces-transplanted F344 rats (PH) and healthy volunteer feces-transplanted F344 rats (HV). A is a diagram showing a comparison of RNA expression in the lungs as a volcano plot. B shows results obtained by performing Gene Ontology Term analysis of RNA expression in the lungs. C is a diagram showing differences in individual gene expression associated with B as a bar plot.

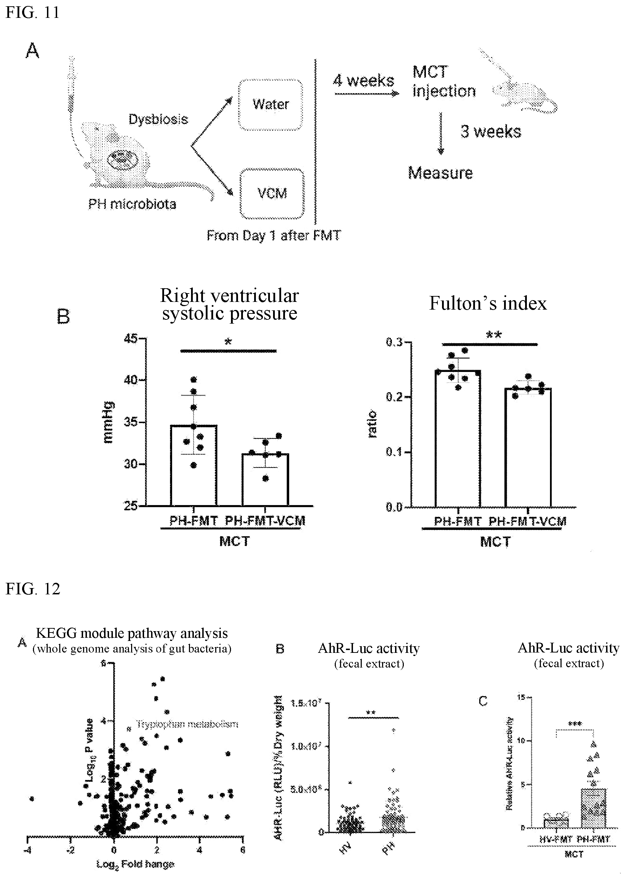

[Fig. 11] Fig. 11 is a diagram showing experimental results obtained by administering vancomycin to PH patient feces-transplanted rats. A is a diagram showing an experimental procedure. B shows results obtained by measuring right ventricular systolic pressure and Fulton's index of PH patient feces-transplanted rats administered vancomycin (PH-FMT-VCM) and PH patient feces-transplanted rats administered water (vehicle) (PH-FMT). The statistical values are mean ± standard deviation. **P < 0.01, *P < 0.05.
[Fig. 12] Fig. 12 is a diagram showing results of a shotgun analysis of gut bacteria using human fecal samples and an AHR luciferase reporter assay using feces of humans and gnotobiotic rats. A is a diagram showing results obtained by performing KEGG module pathway analysis of fecal samples of PH patients and healthy volunteers using shotgun metagenomic data as a volcano plot. B is a diagram showing the results obtained by measuring AHR ligand activity in fecal samples of PH patients (PH) and healthy volunteers (HV) by an AHR luciferase reporter assay. C is a diagram showing results obtained by measuring AHR ligand activity in feces of PH model gnotobiotic rats (PH patient feces-transplanted F344 rats (PH-FMT) and healthy volunteer feces-transplanted F344 rats (HV-FMT)) by an AHR luciferase reporter assay. The statistical values are mean ± standard error. **P < 0.01, ***P < 0.001.

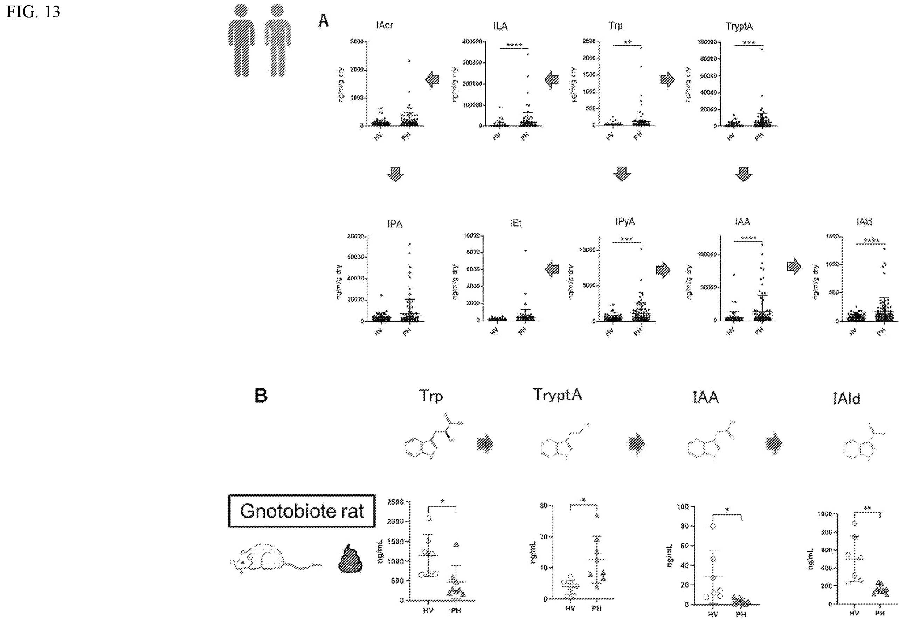

[Fig. 13] Fig. 13 is a diagram showing results of measuring concentrations of tryptophan and metabolites thereof in feces or fecal samples of humans and gnotobiotic rats by targeted LC-MS analysis. A is a diagram showing results obtained by measuring concentrations of tryptophan (Trp) and metabolites thereof (tryptamine (TryptA), indole-3-pyruvic acid (IPyA), indole-3-aldehyde (IAld), indole-3-acetic acid (IAA), indole-3-ethanol (IEt), indole-3-propionic acid (IPA), indole-3-lactic acid (ILA), and indole-3-acrylic acid (IAcr)) in fecal samples of PH patients (PH) and healthy volunteers (HV). B is a diagram showing results obtained by measuring concentrations of tryptophan and metabolites thereof in fecal samples of PH model gnotobiotic rats (PH patient feces-transplanted F344 rats (PH) and healthy volunteer feces-transplanted F344 rats (HV)). The statistical values are mean ± standard deviation. *P < 0.05, **P < 0.01, ***P < 0.001, **** < 0.0001.

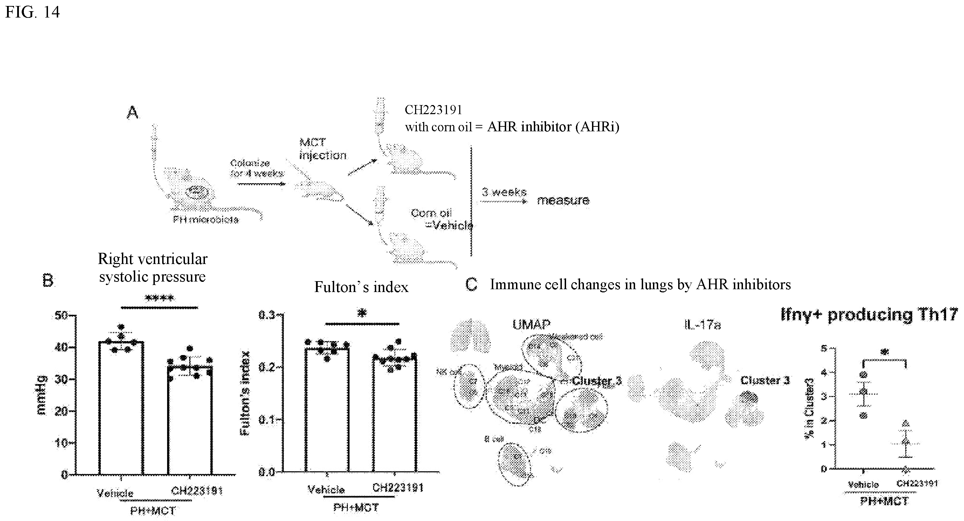

[Fig. 14] Fig. 14 is a diagram showing results of an experiment of administering an AHR inhibitor (CH223191) to PH patient feces-transplanted rats. A is a diagram showing an experimental procedure. B shows results obtained by measuring right ventricular systolic pressure and Fulton's index of PH patient feces-transplanted rats administered an AHR inhibitor (CH223191) or corn oil (vehicle). C is a diagram showing Uniform Manifold Approximation and Projection (UMAP) generated based on results obtained by performing single-cell RNA-seq analysis on immune cells collected from the lungs of the PH patient feces-transplanted rats, and the analysis results thereof. The statistical values are mean ± standard deviation. *P < 0.05, ****P < 0.0001.

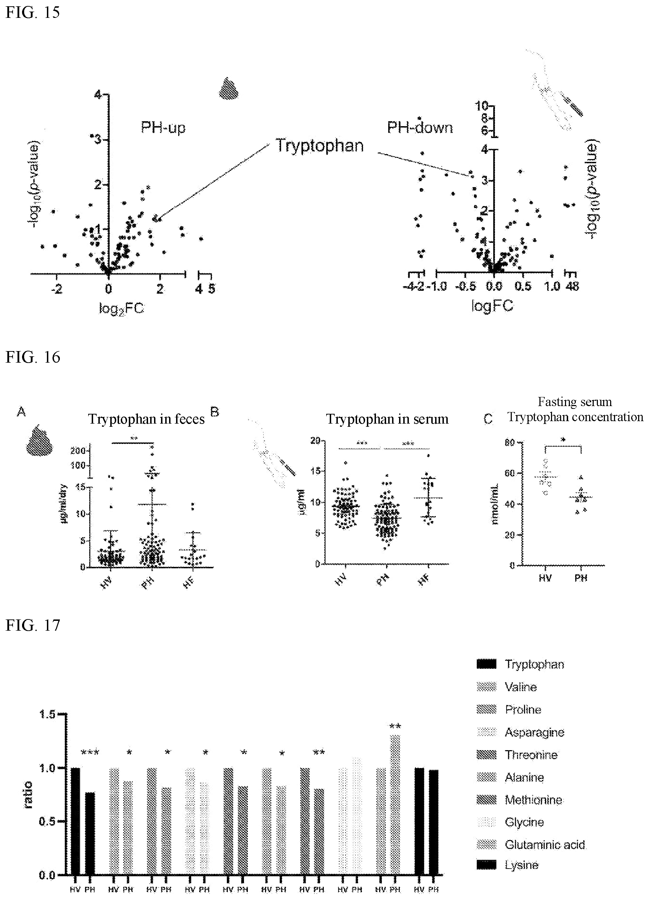

[Fig. 15] Fig. 15 shows results obtained by performing a multi-component simultaneous LC-MS analysis on fecal samples (HV n = 28, PH n = 31) and serum samples (HV n = 24, PH n = 24) of healthy volunteers (HV) and PH patients (PH). The left diagram shows results of fecal samples, and the right diagram shows results of serum samples.
[Fig. 16] Fig. 16 is a diagram showing tryptophan concentrations in fecal samples and serum samples of healthy volunteers (HV), PH patients (PH), and heart failure patients (HF). A shows measurement results of the tryptophan concentration in fecal samples, B shows measurement results of the tryptophan concentration in serum samples, and C shows measurement results of the tryptophan concentration in fasting serum samples. The statistical values are mean ± standard deviation (A, B) or standard error (C). *P < 0.05, **P < 0.01, ***P < 0.001.
[Fig. 17] Fig. 17 is a diagram showing results obtained by measuring amino acid concentrations in serum samples of healthy volunteers and PH patients by LC-MS. In Fig. 17, from a left side, concentrations in serum samples of healthy volunteers (HV) and PH patients (PH) are shown in the order of tryptophan, valine, proline, asparagine, threonine, alanine, methionine, glycine, glutamic acid, and lysine. *P < 0.05, **P < 0.01, ***P < 0.001.

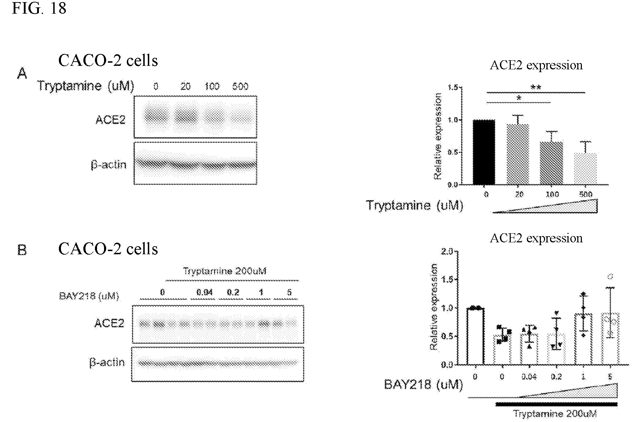

[Fig. 18] Fig. 18 is a diagram showing an in vitro experiment using CACO-2 cells, which are an intestinal epithelial cell line. A is a diagram showing an expression level of an ACE2 protein when cultured by adding tryptamine at 0 to 500 µM. B is a diagram showing an expression level of an ACE2 protein when cultured by adding tryptamine at 200 µM and an AHR inhibitor (BAY-218) at 0 to 5 µM. *P < 0.05, **P < 0.01.

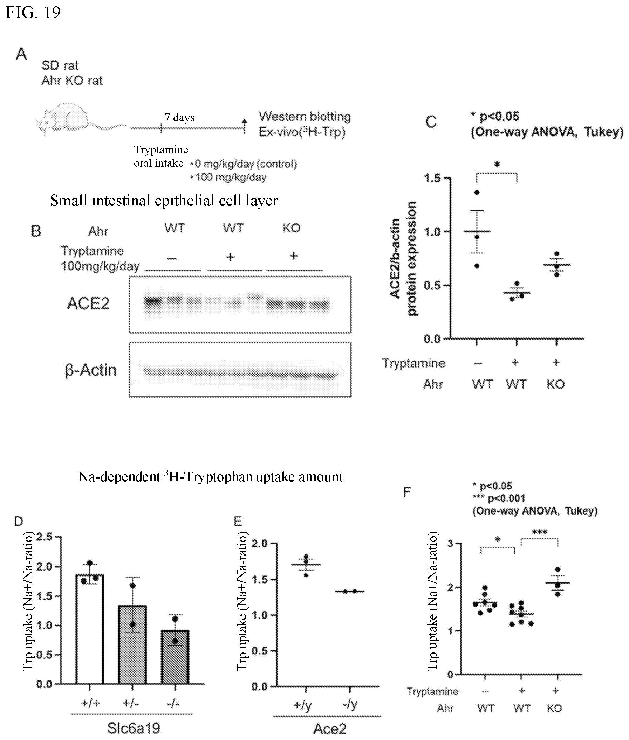

[Fig. 19] Fig. 19 is a diagram showing that tryptamine suppresses ACE2 expression in an AHR-dependent manner and decreases absorption of tryptophan in the small intestine. A is a diagram showing an experimental procedure. B is a diagram showing Western blotting performed using a small intestinal epithelial cell layer. C is a diagram showing quantification of an Ace2 expression level from results of B. D shows results obtained by performing an uptake experiment of tritium-labeled tryptophan on the small intestine excised from wild-type SD rats (Slc6a19 +/+) and Slc6a19 knockout rats (Slc6a19 +/-, -/-). E shows results obtained by performing an uptake experiment of tritium-labeled tryptophan on the small intestine excised from wild-type SD rats (Ace2 +/y) and Ace2 knockout rats (Ace2 -/y). The statistical values are mean ± standard error. *P < 0.05, ***P < 0.001.

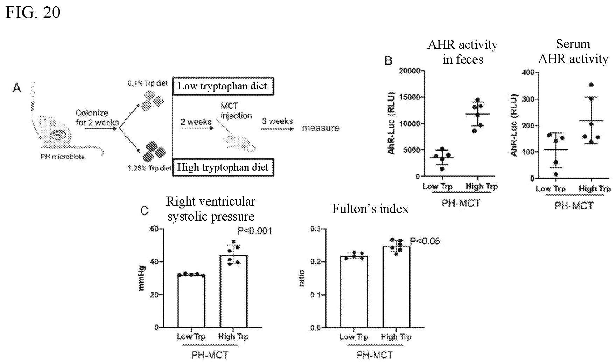

[Fig. 20] Fig. 20 is a diagram showing that tryptophan is important for the pathogenesis of PH. A is a diagram showing a protocol of an experiment. B is a diagram showing results obtained by measuring the AHR activity in feces and serum samples. C shows results obtained by measuring right ventricular systolic pressure and Fulton's index. The statistical values are mean ± standard deviation. D shows results obtained by measuring the concentration of tryptophan metabolites in feces. **P < 0.01, ***P < 0.001, **** < 0.0001.

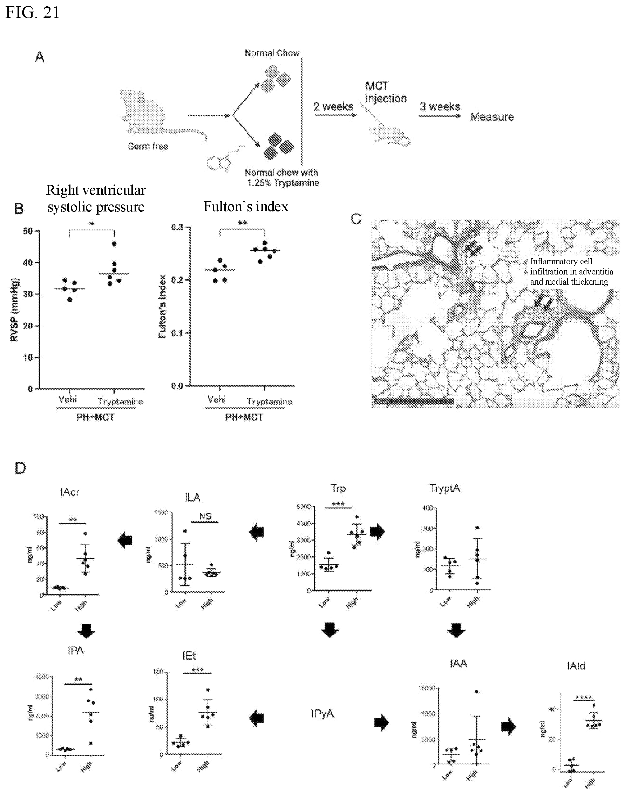

[Fig. 21] Fig. 21 is a diagram showing that intake of tryptamine induces exacerbation of PH pathology. A is a diagram showing an experimental procedure. B shows results obtained by measuring right ventricular systolic pressure and Fulton's index. C shows Elastica van Gieson-stained images of lungs of rats fed a tryptamine-containing diet. In C, arrows are attached to sites where infiltration of inflammatory cells is observed. The statistical values are mean ± standard deviation. *P < 0.05, **P < 0.01.

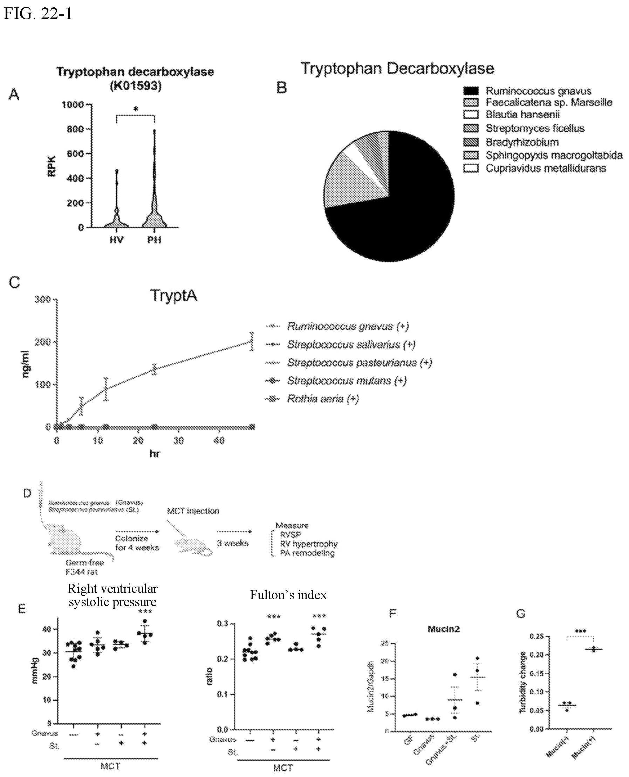

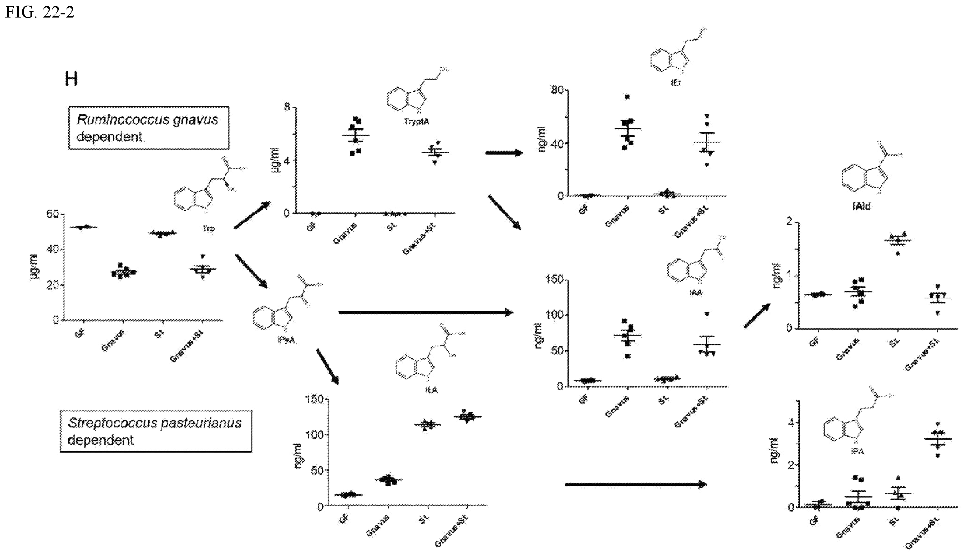

[Fig. 22] Fig. 22 is a diagram showing that Ruminococcus gnavus exacerbates PH through a cooperative effect with Streptococcus pasteurianus. A shows results obtained by performing whole genome analysis (shotgun analysis) using fecal samples of healthy volunteers (HV) and PH patients (PH) and determining a prevalence of a gene (K01593) encoding tryptophan decarboxylase. B shows results obtained by analyzing species proportions of bacteria carrying the K01593 gene. C shows results obtained by anaerobically culturing Ruminococcus gnavus, Streptococcus salivarius, Streptococcus pasteurianus, and Streptococcus mutans in vitro and measuring the concentration of tryptamine (TryptA) in a culture medium over time. D is a diagram showing a protocol of an administration experiment of Ruminococcus gnavus and Streptococcus pasteurianus to germ-free rats. E shows results obtained by measuring right ventricular systolic pressure and Fulton's index in the experiment of D. F shows results obtained by measuring a mucin expression level in small intestinal epithelial cells by qPCR in the experiment of D. In F, "GF" indicates a condition without bacterial inoculation, "Gnavus" indicates a condition inoculated with Ruminococcus gnavus alone, "Gnavus+St" indicates a condition inoculated with both Ruminococcus gnavus and Streptococcus pasteurianus, and "St" indicates a condition inoculated with Streptococcus pasteurianus alone. G shows results obtained by performing a culture experiment of Ruminococcus gnavus in the presence or absence of mucin and measuring turbidity of a culture medium after culture. H shows results obtained by measuring concentrations of tryptophan and metabolites thereof in feces by LC-MS in the experiment of D. In H, "GF" indicates a condition without bacterial inoculation, "Gnavus" indicates a condition inoculated with Ruminococcus gnavus alone, "St" indicates a condition inoculated with Streptococcus pasteurianus alone, and "Gnavus+St" indicates a condition inoculated with both Ruminococcus gnavus and Streptococcus pasteurianus. The statistical values are mean ± standard deviation. *P < 0.05, ***P < 0.001.

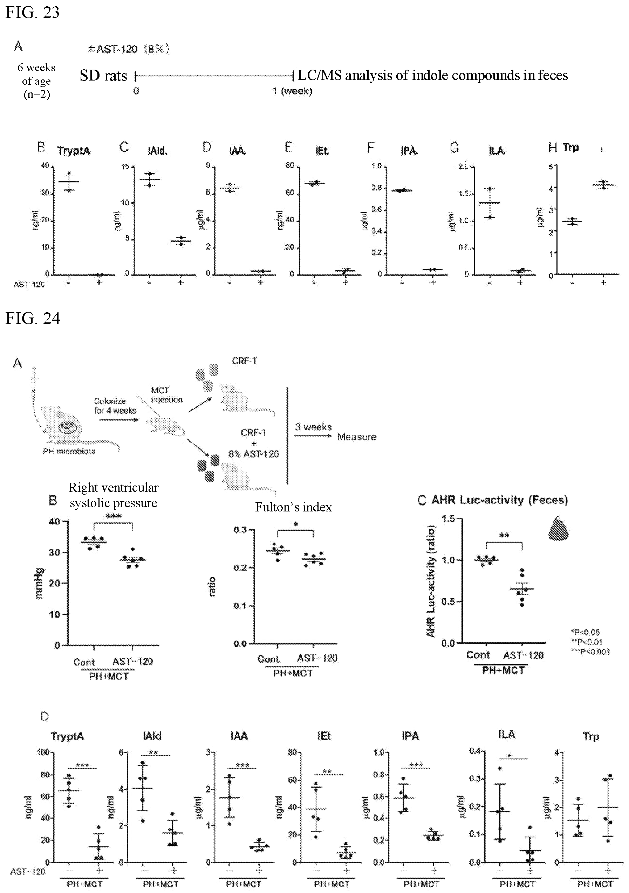

[Fig. 23] Fig. 23 is a diagram showing that dietary administration of a carbonaceous adsorbent AST-120 adsorbs and removes various indole compounds from the intestine of rats. A is a diagram showing a protocol of an experiment. B shows the concentrations of tryptamine (TryptA) and indole-3-pyruvic acid (IPyA) in feces; C shows the concentration of indole-3-aldehyde (IAld) in feces; D shows the concentration of indole-3-acetic acid (IAA) in feces; E shows the concentration of indole-3-ethanol (IEt) in feces; F shows the concentration of indole-3-propionic acid (IPA) in feces; G shows the concentration of indole-3-lactic acid (ILA) in feces; and H shows the concentration of tryptophan (Trp) in feces.
[Fig. 24] Fig. 24 shows results indicating that, in PH model gnotobiotic rats, oral administration of AST-120 removed tryptophan metabolites in the intestine and significantly improved PH pathology. A is a diagram showing a protocol of an experiment. B shows results obtained by measuring right ventricular systolic pressure and Fulton's index. C shows results obtained by measuring AHR ligand activity in feces. D shows results obtained by measuring respective concentrations of tryptamine (TryptA), indole-3-pyruvic acid (IPyA), indole-3-aldehyde (IAld), indole-3-acetic acid (IAA), indole-3-ethanol (IEt), indole-3-propionic acid (IPA), indole-3-lactic acid (ILA), and tryptophan (Trp) in feces. The statistical values are mean ± standard deviation. *P < 0.05, **P < 0.01, ***P < 0.001.

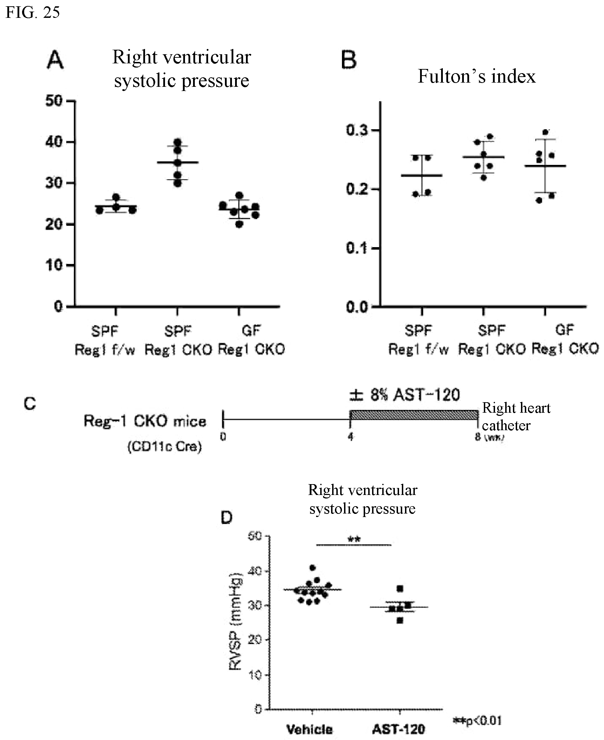

[Fig. 25] Fig. 25 is a diagram showing that spontaneous onset of PAH is suppressed when Regnase-1 Cd11cCre (Reg-1 CKO) mice, which are a severe PAH spontaneous onset model, are housed in a germ-free environment, and that the pathogenesis of PAH is suppressed when a carbonaceous adsorbent is administered to the Reg-1 CKO mice. A and B show results obtained by measuring right ventricular systolic pressure (A) and Fulton's index (B) after housing Reg-1 CKO mice in an SPF environment or a germ-free (GF) environment. In A, Reg1 f/w is a control group in which PAH does not develop. C shows a protocol of an experiment in which AST-120 was administered to Reg-1 CKO mice. D shows results obtained by measuring right ventricular systolic pressure in the experiment of C. The statistical values are mean ± standard deviation. **P < 0.01.

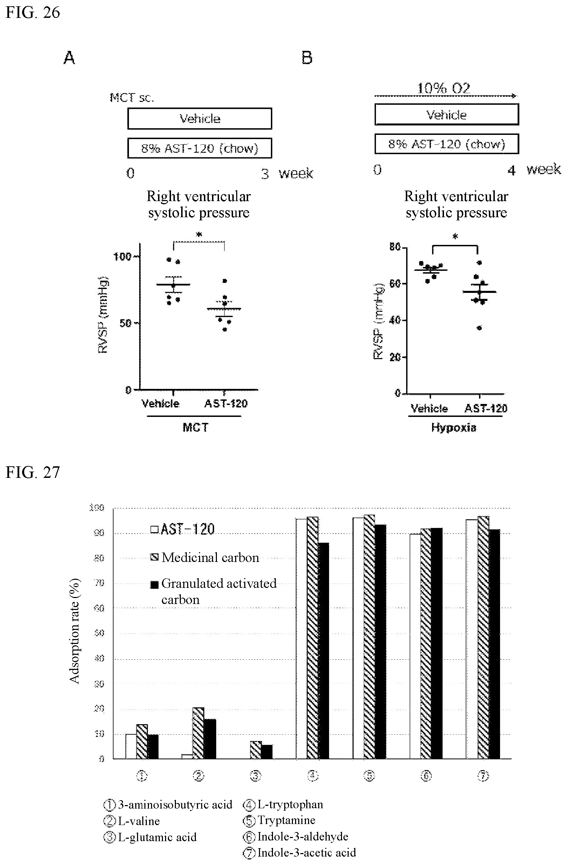

[Fig. 26] Fig. 26 is a diagram showing that the pathogenesis of PAH is suppressed when a carbonaceous adsorbent is administered to PH model rats. A shows results obtained by measuring right ventricular systolic pressure after administering AST-120 to monocrotaline-loaded PH model rats. B shows results obtained by measuring right ventricular systolic pressure after administering AST-120 to hypoxia-loaded PH model rats. *P < 0.05.
[Fig. 27] Fig. 27 is a diagram showing that a carbonaceous adsorbent (AST-120, medicinal carbon, and granulated activated carbon) efficiently binds to indole compounds. Results obtained by measuring the adsorption rates of each carbonaceous adsorbent for L-valine, L-glutamic acid, 3-aminoisobutyric acid, L-tryptophan, tryptamine, indole-3-aldehyde, and indole-3-acetic acid are shown.

### Description of Embodiments

### 1. Definitions

Unless specifically defined otherwise, the terms used herein have the meanings as commonly understood by one of ordinary skill in the art of medicine, pharmacy, molecular biology, microbiology, organic chemistry, and the like. Terms defined in this specification shall, when they do not have the same meanings as generally understood, be governed by the descriptions of this specification.

In the present disclosure, "pulmonary hypertension (PH)" is a progressive disease group with poor prognosis in which the increase in blood pressure in the pulmonary artery leads to impairment of functions of the heart and the lung, and specifically, "pulmonary hypertension (PH)" is a medical condition in which the mean value (mean PAP) of pulmonary arterial pressure (PAP) measured using right heart catheterization at rest is 25 mmHg or more. PH is classified, according to the Nice classification, into Group 1: pulmonary arterial hypertension (PAH); Group 1': pulmonary veno-occlusive disease (PVOD) and/or pulmonary capillary hemangiomatosis (PCH); Group 1'': persistent pulmonary hypertension of a newborn; Group 2: pulmonary hypertension associated with a left heart disease; Group 3: pulmonary hypertension associated with a lung disease and/or hypoxemia; Group 4: chronic thromboembolic pulmonary hypertension; and Group 5: pulmonary hypertension associated with a multifactorial mechanism of unknown details.

In the present disclosure, "severity of PH" is classified according to the WHO Functional Classification for pulmonary hypertension shown in Table 1.

**[Table 1]**

| WHO Functional Classification for pulmonary hypertension | |
|---|---|
| Class I | Pulmonary hypertension patients without limitation in physical activity. |
| | Ordinary physical activity does not cause dyspnea, fatigue, chest pain, presyncope, or the like. |
| Class II | Pulmonary hypertension patients with slight limitation in physical activity. |
| | There are no subjective symptoms at rest. Ordinary physical activity causes dyspnea, fatigue, chest pain, presyncope, or the like. |
| Class III | Pulmonary hypertension patients with marked limitation in physical activity. |
| | There are no subjective symptoms at rest. Less than ordinary, slight physical activity causes dyspnea, fatigue, chest pain, presyncope, or the like. |
| Class IV | Pulmonary hypertension patients for whom any physical activity causes discomfort. |
| | Some of these patients present with symptoms of right heart failure. |
| | Dyspnea and/or fatigue is also present at rest. Exacerbation of subjective symptoms occurs with any physical activity. |

In the present disclosure, the "aryl hydrocarbon receptor (AHR)" refers to a transcription factor that is activated by binding to an agonist (endogenous or exogenous ligand) to control downstream gene expression such as CYP1A1 (Cytochrome P450, Family 1, Subfamily A) and AHRR (AHR repressor).

In the present disclosure, "B0AT1" is a sodium-dependent neutral amino acid transporter B(0)AT1, and is a protein encoded by the SLC6A19 gene.

In the present disclosure, "angiotensin-converting enzyme 2 (ACE2)" is one of the factors that act in the renin-angiotensin system, and is a protein that is also known to contribute to neutral amino acid absorption in the intestinal tract by functioning as a chaperone of B0AT1.

In the present disclosure, the "indole compounds that are tryptophan metabolites" refer to compounds having an indole ring among compounds produced by metabolism of tryptophan. Specific examples of the indole compounds that are tryptophan metabolites include tryptamine (TryptA), indole-3-aldehyde (IAld), indole-3-pyruvic acid (IPyA), indole-3-ethanol (IEt), indole-3-acetic acid (IAA), indole-3-propionic acid (IPA), indole-3-acrylic acid (IAcr), indole-3-lactic acid (ILA), indole-3-acetaldehyde, indole-3-acetamide, indole-3-acetonitrile, indoxyl sulfate, 2-oxindole, indole-3-butyric acid, 3-methylindole, N-methyltryptamine, N-hydroxytryptamine, 3-methylindolepyruvic acid, indole-3-acetaldoxime, 3-methyldeoxyindole, 2-oxindoleacetic acid, 2-oxindole, indole-3-carbinol, indole, and indole-3-glycerol phosphate.

In the present disclosure, "prevention" refers to suppressing the onset of a disease or symptom or prolonging the period until the onset, and "treatment" refers to reduction, alleviation, or decrease in rate of progression of a disease or symptom.

In the present disclosure, a "blood sample" is a sample derived from blood, and includes whole blood, serum, and plasma.

### 2. Prophylactic or Therapeutic Agent for PH Based on Mechanism of Removing Tryptophan Metabolites in intestinal Tract (1)

The present inventors have identified that indole compounds that are tryptophan metabolites are ligands of AHR that plays an important role in the pathogenesis of PH, and have found that PH can be prevented or treated by removing the indole compound from the intestinal tract. Therefore, in one embodiment of the present disclosure, there is provided a prophylactic or therapeutic agent for PH (hereinafter referred to as "PH prophylactic or therapeutic agent (1)") containing a substance capable of removing indole compounds that are tryptophan metabolites from the intestinal tract. Hereinafter, the PH prophylactic or therapeutic agent (1) of the present disclosure will be described in detail.

### [Active Ingredients]

In the PH prophylactic or therapeutic agent (1) of the present disclosure, a substance capable of removing indole compounds that are tryptophan metabolites from the intestinal tract is contained as an active ingredient. In the PH prophylactic or therapeutic agent (1) of the present disclosure, PH can be prevented or treated by removal of tryptophan metabolites (indole compounds) present in the intestinal tract.

In the PH prophylactic or therapeutic agent (1) of the present disclosure, the type of the indole compound to be removed from the intestinal tract is not particularly limited, and preferable examples thereof include tryptamine, indole-3-aldehyde, indole-3-pyruvic acid, indole-3-ethanol, indole-3-acetic acid, indole-3-propionic acid, indole-3-acrylic acid, and indole-3-lactic acid. The substance used as an active ingredient may be a substance capable of removing one type of indole compound from the intestinal tract, but a substance capable of removing two or more types of indole compounds from the intestinal tract is preferable. Among the indole compounds, tryptamine plays a particularly important role in the pathogenesis of PH, and therefore, the substance used as an active ingredient is preferably a substancethat can remove at least tryptamine from the intestinal tract. In addition, since tryptophan is an essential amino acid, preferably, the substance used as an active ingredient is difficult to remove tryptophan from the intestinal tract.

The type of the substance capable of removing the indole compounds that are tryptophan metabolites from the intestinal tract is not particularly limited, and specific examples thereof include a substance that adsorbs or binds to the indole compounds that are tryptophan metabolites. According to the substance, it is possible to prevent or treat PH by adsorbing or binding to tryptophan metabolites (indole compounds) present in the intestinal tract and discharging the tryptophan metabolite as feces without causing the tryptophan metabolite to function as an AHR ligand.

The substance that adsorbs to the indole compounds is not particularly limited as long as the substance interferes with the function as an AHR ligand by being adsorbed to the indole compounds, and a suitable example thereof preferably include a porous inorganic adsorbent (inorganic adsorbent). Specific examples of the porous inorganic adsorbent include a carbonaceous adsorbent (activated carbon, medicinal carbon, and the like), activated alumina, silica gel, bentonite, diatomaceous earth, zeolite, molecular sieve, white clay, silica-magnesia preparations, silica, zinc aluminosilicate, titanium oxide, iron oxide, tin oxide, titanium hydroxide, iron hydroxide, tin hydroxide, calcium carbide, montmorillonite, hydroxyapatite, talc, kaolin, carbon nanotubes, aluminum silicate, magnesium silicate, hydrotalcite, magnesium aluminometasilicate, aluminum oxide, aluminum oxide, aluminum hydroxide gel, silica-alumina-based minerals (for example, Sivlet, Secard), carbon nitride, platinum, glass, and pumice. The pore diameter of a porous inorganic adsorbent is not particularly limited, and the porous inorganic adsorbent may have any of micropores (2 nm or less), mesopores (2 to 50 nm), or macropores (50 nm or more), but a suitable example of the porous inorganic adsorbent includes a porous inorganic adsorbent having mesopores (a mesoporous inorganic adsorbent). In addition, the shape of the porous inorganic adsorbent is not particularly limited as long as the porous inorganic adsorbent can be administered into a living body, and the porous inorganic adsorbent may be any of a powder, a granulated product, and the like.

Further, other examples of the substance that adsorbs indole compounds include metal-organic frameworks (porous coordination polymers composed of metal ions and bridging organic ligands); tannic acid-based adsorbents such as tannic acid albumin; protein-based adsorbents such as albumin, lactoferrin, lactoglobulin, casein, and fragments thereof; thickeners such as gelatin, agar, alginic acid, carrageenan, pectin, and dextrin; micelles such as casein micelles; emulsifiers; synthetic adsorbents having, as a base polymer, polydivinylbenzene, polymethacryl, polymethacryl-divinylbenzene crosslinked polymers, polystyrene-divinylbenzene crosslinked polymers, and the like; ion exchange resins; and analogous structures thereof.

Among the substances that adsorb indole compounds, a suitable example includes a porous inorganic adsorbent (an inorganic adsorbent), and a more preferable example includes a carbonaceous adsorbent. Conventionally, spherical carbonaceous adsorbent for oral administration, such as spherical carbonaceous adsorbent AST-120 (trade name: Kremezin), spherical carbonaceous adsorbent (trade name: Mylan), and spherical carbonaceous adsorbent (trade name: Nichi-Iko), has been clinically used for the purpose of improving uremic symptoms in chronic renal failure and delaying initiation of dialysis, and in the present disclosure, AST-120, spherical carbonaceous adsorbent (Mylan), or spherical carbonaceous adsorbent (Nichi-Iko) can also be used as the carbonaceous adsorbent.

As a substance that binds to indole compounds, the substance is not particularly limited as long as the substance binds to indole compounds and thereby inhibits a function thereof as an AHR ligand, and specific examples thereof include an antibody against an indole compound and a fragment thereof; albumin (a recombinant albumin protein, and the like), a fragment thereof, a partial peptide thereof (including a recombinant form, and the like), and analogous substances thereof; an AHR protein (a recombinant AHR protein, and the like), a partial peptide thereof (including a recombinant form, and the like), and analogous substances thereof; an F-box protein (TIR1), which is a subunit of an auxin (indole-3-acetic acid) receptor SCF (Skp1/Cullin/F-box) complex (a recombinant TIR1 protein, and the like), a partial peptide thereof (including a recombinant form, and the like), and analogous substances thereof; a serotonin receptor 4 (a recombinant serotonin receptor 4 protein, and the like), a partial peptide thereof (including a recombinant form, and the like), and analogous substances thereof; other indole compound-binding proteins (including recombinant forms, and the like), partial peptides thereof, and analogous substances thereof. In addition, albumin has not only a property of binding to tryptamine but also a property of adsorbing to indole compounds, and therefore albumin, a fragment thereof, a partial peptide thereof, and the like can be used as either a substance that adsorbs to indole compounds or a substance that binds to indole compounds.

In one embodiment of the PH prophylactic or therapeutic agent (1) of the present disclosure, examples of the active ingredient preferably include a substance that adsorbs to indole compounds, more preferably an inorganic adsorbent (porous inorganic adsorbent), further preferably a carbonaceous adsorbent, particularly preferably spherical carbonaceous adsorbent, and still more preferably AST-120, spherical carbonaceous adsorbent Mylan and spherical carbonaceous adsorbent Nichi-Iko.

In the PH prophylactic or therapeutic agent (1) of the present disclosure, the substance capable of removing the indole compounds that are tryptophan metabolites from the intestinal tract may be used alone, or may be used in combination with two or more thereof.

### [Formulation]

The PH prophylactic or therapeutic agent (1) of the present disclosure is provided by being prepared into a desired dosage form by blending the active ingredient with a pharmaceutically acceptable carrier, additive, or the like. Examples of pharmaceutically acceptable carriers or additives include sterile water, physiological saline, stabilizers, excipients, antioxidants, buffers, preservatives, surfactants, chelating agents, and binders.

Examples of the dosage form of the PH prophylactic or therapeutic agent (1) of the present disclosure include capsules, tablets, pills, sachets, liquids, powders, granules, fine granules, film-coated agents, pellets, troches, sublingual tablets, chewable tablets, buccal tablets, pastes, syrups, suspensions, elixirs, or emulsions. The content of the active ingredient in the PH prophylactic or therapeutic agent (1) of the present disclosure may be appropriately set according to the dose, dosage form, and the like.

### [Dosage and Administration]

In the PH prophylactic or therapeutic agent (1) of the present disclosure, the clinical classification of PH to be prevented or treated is not particularly limited, and the PH may be classified into any one of Group 1, Group 1', Group 1", Group 2, Group 3, Group 4, and Group 5 of the Nice classification. As one embodiment of the PH prophylactic or therapeutic agent (1) of the present disclosure, the PH prophylactic or therapeutic agent (1) can be used for preventing or treating Group 1 (PAH) of the Nice classification.

In addition, in the PH prophylactic or therapeutic agent (1) of the present disclosure, the severity of PH to be treated is not particularly limited, and may be any of the severity of Class I to Class IV in the WHO Functional Classification for pulmonary hypertension.

In the PH prophylactic or therapeutic agent (1) of the present disclosure, an administration route is not particularly limited as long as the active ingredient can be transferred into the intestine, and examples thereof include oral administration and enteral administration, and preferably oral administration.

The dose of the PH prophylactic or therapeutic agent (1) of the present disclosure may be appropriately set according to the age of the subject to be administered, the degree of PH pathology, the type of the active ingredient to be used, and the like, and for example, the active ingredient may be administered at approximately 1 to 80000 mg/kg per day for an adult, preferably approximately 10 to 8000 mg/kg per day, in 1 to 6 divided doses per day, preferably in 2 to 4 divided doses per day. More specifically, when a porous inorganic adsorbent (particularly carbonaceous adsorbent) is used as the active ingredient, the porous inorganic adsorbent may be administered at approximately 10 to 8000 mg/kg per day for an adult, preferably approximately 80 to 500 mg/kg per day, in 1 to 6 divided doses per day, preferably in 2 to 4 divided doses per day.

### 3. Method for Examining Presence or Absence of PH or Severity of PH Using Tryptophan Concentrations in Feces, Gastrointestinal Contents, or Blood Samples as Indicator (PH Examination Method (1))

The present inventors have found that inhibition of absorption of tryptophan occurs in the intestinal tract of PH patients, and that PH patients have higher concentrations of tryptophan in feces and gastrointestinal contents and lower concentrations of tryptophan in blood as compared with healthy volunteers. Therefore, in one embodiment of the present disclosure, there is provided a method (hereinafter described as "PH examination method (1)") for examining the presence or absence of PH or the severity of PH, the examination method including a step of measuring the tryptophan concentrations in feces, gastrointestinal contents, or blood samples collected from a subject. Hereinafter, the PH examination method (1) of the present disclosure will be described in detail.

In the PH diagnosis method (1) of the present disclosure, the subject may be a person who needs to be examined for the presence or absence of PH or a person who needs to be examined for the severity of PH. Examples of the person who needs to be examined for the presence or absence of PH include a healthy volunteer and a person suspected of having onset of PH. Examples of the person who needs to be examined for the severity of PH include a PH patient, a person suspected of having onset of PH, and the like.

In the PH examination method (1) of the present disclosure, the biological sample of which the tryptophan concentration is measured may be any of feces, gastrointestinal contents, and blood samples. The gastrointestinal contents are preferably contents in the small intestine. The gastrointestinal contents can be collected by, for example, nasal intestinal insertion or the like. Feces are preferable because they can be collected non-invasively. When feces or gastrointestinal contents are used, an extract containing tryptophan may be prepared from the feces or gastrointestinal contents using an extraction solvent such as water as a sample for measuring the tryptophan concentration. The blood sample may be any of whole blood, serum, or plasma, and a suitable example thereof includes serum.

The tryptophan concentrations in feces, gastrointestinal contents, or blood samples can be measured by a known tryptophan measurement method such as high performance liquid chromatography, liquid chromatography mass spectrometry, gas chromatography mass spectrometry, or enzyme immunoassay (ELISA).

When the presence or absence of PH is examined in the PH examination method (1) of the present disclosure, a subject having high tryptophan concentrations in feces or gastrointestinal contents is determined to have a high likelihood of having PH. In the examination of the presence or absence of PH, the level of tryptophan concentrations in feces or gastrointestinal contents can be determined by obtaining in advance, as a reference value, the mean value or median value of the tryptophan concentrations in feces or gastrointestinal contents in healthy volunteers and/or PH patients, or the cutoff value for distinguishing between the healthy volunteers and the PH patients, and comparing the measured value with the reference value. For example, when the tryptophan concentrations in feces or gastrointestinal contents in a subject are higher than the mean value or median value of the tryptophan concentrations in feces or gastrointestinal contents in healthy volunteers, it can be determined that the subject is highly likely to suffer from PH. In addition, when the tryptophan concentrations in feces or gastrointestinal contents in a subject are equal to or higher than the mean value or median value of the tryptophan concentrations in feces or gastrointestinal contents in PH patients, it can be determined that the subject is likely to suffer from PH. From the experimental results described later, regarding the tryptophan concentrations in feces, it has been confirmed that the mean value for PH patients is 113.7 µg/g (dry fecal wt.), the median value for PH patients is 28.9 µg/g (dry fecal wt.), and the cutoff value at which the sensitivity and specificity are optimized between PH patients and healthy volunteers is 26.18 µg/g (dry fecal wt.). Therefore, any of these values can be used as a reference value to determine the presence or absence of PH in a subject.

In addition, when the presence or absence of PH is examined in the PH examination method (1) of the present disclosure, a subject having low tryptophan concentrations in blood samples is determined to have a high likelihood of having PH. In the examination of the presence or absence of PH, the level of tryptophan concentrations in blood samples can be determined by obtaining in advance, as a reference value, the mean value or median value of the tryptophan concentrations in blood samples in healthy volunteers and/or PH patients, or the cutoff value for distinguishing between the healthy volunteers and the PH patients, and comparing the measured value with the reference value. For example, when the tryptophan concentrations in blood samples in a subject are lower than the mean value or median value of the tryptophan concentrations in blood samples in healthy volunteers, it can be determined that the subject is highly likely to suffer from PH. In addition, when the tryptophan concentrations in blood samples in a subject are equal to or lower than the mean value or median value of the tryptophan concentrations in blood samples in PH patients, it can be determined that the subject is highly likely to suffer from PH. From the experimental results described later, regarding the tryptophan concentrations in the serum, it has been confirmed that the mean value of PH patients is 7.48 µg/mL, the median value of PH patients is 7.35 µg/mL, and the cutoff value at which the sensitivity and specificity are optimized between PH patients and healthy volunteers is 13.0 µg/mL or less. Therefore, any of these values can be used as a reference value to determine the presence or absence of PH in a subject.

In addition, when the severity of PH is examined in the PH examination method (1) of the present disclosure, a subject having higher tryptophan concentrations in feces or gastrointestinal contents is determined to be more likely to have a higher severity of PH. In examination of the severity of PH, the level of tryptophan concentrations in feces or gastrointestinal contents can be determined by dividing PH patients according to severity as a reference value in advance, obtaining the mean value or median value of tryptophan concentrations in feces or gastrointestinal contents, or the cutoff value for distinguishing between different severity levels, and comparing the measured value with the reference value.

In addition, when the severity of PH is examined in the PH examination method (1) of the present disclosure, a subject having lower tryptophan concentrations in blood samples is determined to be more likely to have a higher severity of PH. In the examination of the severity of PH, the level of tryptophan concentrations in blood samples can be determined by dividing PH patients according to severity as a reference value in advance, obtaining the mean value or median value of tryptophan concentrations in blood samples, or the cutoff value for distinguishing between different severity levels, and comparing the measured value with the reference value.

In the PH examination method (1) of the present disclosure, when the tryptophan concentrations in feces or gastrointestinal contents are high and the tryptophan concentrations in blood samples are low, a subject is determined to have a high likelihood of having PH or a potentially high severity of PH with higher accuracy than when determined by only one of the indicators.

Since the PH examination method (1) of the present disclosure can be performed as an examination for assisting diagnosis of PH, for a subject who is determined to have a high likelihood of having PH or having a likelihood of a high severity of PH by the PH examination method (1) of the present disclosure, it is desirable to perform further examinations such as catheterization, echocardiography, electrocardiography, and chest X-ray examination also for a definitive diagnosis.

In addition, in one aspect of the present disclosure, an examination kit used for performing the PH examination method (1) is provided. The examination kit may contain a reagent for measuring tryptophan. In addition, the examination kit may further contain an instrument for collecting feces or gastrointestinal contents, a reagent for extracting tryptophan from feces or gastrointestinal contents, and the like as necessary.

### 4. Method for Examining Presence or Absence of PH or Severity of PH Using Tryptophan Metabolite Concentrations in Feces, Gastrointestinal Contents, or Blood Samples as Indicator (PH Examination Method (2))

The present inventors have found that, due to high tryptophan concentrations in the intestinal tract of PH patients, concentrations of indole compounds (AHR ligands) that are tryptophan metabolites are high in the gut microbiota and blood. Therefore, in one embodiment of the present disclosure, there is provided a method (hereinafter described as "PH examination method (2)") for examining the presence or absence of PH or the severity of PH, the examination method including a step of measuring the concentrations of indole compounds that are tryptophan metabolites in feces, gastrointestinal contents, or blood samples collected from a subject. Hereinafter, the PH examination method (2) of the present disclosure will be described in detail.

In the PH examination method (2) of the present disclosure, the subject, feces, gastrointestinal contents, and the blood samples are the same as those in the PH examination method (1).

In the PH examination method (2) of the present disclosure, the indole compound to be measured may be at least one selected from the group consisting of tryptamine, indole-3-aldehyde, indole-3-pyruvic acid, indole-3-ethanol, indole-3-acetic acid, indole-3-propionic acid, indole-3-acrylic acid, and indole-3-lactic acid. Among these indole compounds, since the tryptamine concentration has a high correlation with the pathology of PH, the indole compound to be measured preferably includes at least tryptamine. When two or more indole compounds are to be measured, the total concentration of the two or more indole compounds may be determined.

The indole compound concentrations in feces, gastrointestinal contents, or blood samples can be measured by a known tryptophan measurement method such as high performance liquid chromatography, liquid chromatography mass spectrometry, gas chromatography mass spectrometry, or enzyme immunoassay (ELISA).

When the presence or absence of PH is examined in the PH examination method (2) of the present disclosure, a subject having high indole compound concentrations in feces, gastrointestinal contents, or blood samples is determined to have a high likelihood of having PH. In the examination of the presence or absence of PH, the level of indole compound concentrations in feces, gastrointestinal contents, or blood samples can be determined by obtaining in advance, as a reference value, the mean value or median value of the indole compound concentrations in feces, gastrointestinal contents, or blood samples in healthy volunteers and/or PH patients, or the cutoff value for distinguishing between the healthy volunteers and the PH patients, and comparing the measured value with the reference value. For example, when the indole compound concentrations in feces, gastrointestinal contents, or blood samples in a subject are higher than the mean value or median value of the indole compound concentrations in feces, gastrointestinal contents, or blood samples in healthy volunteers, it can be determined that the subject is likely to suffer from PH. In addition, when the indole compound concentrations in feces, gastrointestinal contents, or blood samples in a subject are equal to or higher than the mean value or median value of the indole compound concentrations in feces, gastrointestinal contents, or blood samples in PH patients, it can be determined that the subject is likely to suffer from PH.

When the presence or absence of PH is examined, specific reference values estimated from experimental results described later are as follows. Regarding tryptamine concentrations in feces, it has been confirmed that the mean value for PH patients is 4648 ng/g (dry fecal wt.), the median value for PH patients is 1589 ng/g (dry fecal wt.), and the cutoff value at which sensitivity and specificity between PH patients and healthy volunteers are optimized is 1559 ng/g (dry fecal wt.). Regarding the indole-3-aldehyde concentrations in feces, it has been confirmed that the mean value for PH patients is 179.9 ng/g (dry fecal wt.), the median value for PH patients is 106.0 ng/g (dry fecal wt.), and the cutoff value at which the sensitivity and specificity are optimized between PH patients and healthy volunteers is 97.13 ng/g (dry fecal wt.). Regarding the indole-3-pyruvic acid concentrations in feces, it has been confirmed that the mean value for PH patients is 1177 ng/g (dry fecal wt.), the median value for PH patients is 623 ng/g (dry fecal wt.), and the cutoff value at which the sensitivity and specificity are optimized between PH patients and healthy volunteers is 558 ng/g (dry fecal wt.). Regarding the indole-3-ethanol concentrations in feces, it has been confirmed that the mean value for PH patients is 363.1 ng/g (dry fecal wt.), the median value for PH patients is 155.9 ng/g (dry fecal wt.), and the cutoff value at which the sensitivity and specificity are optimized between PH patients and healthy volunteers is 342.6 ng/g (dry fecal wt.). Regarding the indole-3-acetic acid concentrations in feces, it has been confirmed that the mean value for PH patients is 13986 ng/g (dry fecal wt.), the median value for PH patients is 4851 ng/g (dry fecal wt.), and the cutoff value at which the sensitivity and specificity are optimized between PH patients and healthy volunteers is 6097 ng/g (dry fecal wt.). Regarding the indole-3-propionic acid concentrations in feces, it has been confirmed that the mean value for PH patients is 7238 ng/g (dry fecal wt.), the median value for PH patients is 2750 ng/g (dry fecal wt.), and the cutoff value at which the sensitivity and specificity are optimized between PH patients and healthy volunteers is 12630.8 ng/g (dry fecal wt.). Regarding the indole-3-acrylic acid concentrations in feces, it has been confirmed that the mean value for PH patients is 174.1 ng/g (dry fecal wt.), the median value for PH patients is 84.5 ng/g (dry fecal wt.), and the cutoff value at which the sensitivity and specificity are optimized between PH patients and healthy volunteers is 159.2 ng/g (dry fecal wt.). Regarding the indole-3-lactic acid concentrations in feces, it has been confirmed that the mean value for PH patients is 20099 ng/g (dry fecal wt.), the median value for PH patients is 830.5 ng/g (dry fecal wt.), and the cutoff value at which the sensitivity and specificity are optimized between PH patients and healthy volunteers is 249.1 ng/g (dry fecal wt.). At least one of these values can also be used as a reference value to determine the presence or absence of PH in a subject.

When the severity of PH is examined in the PH examination method (2) of the present disclosure, a subject having higher indole compound concentration in feces, gastrointestinal contents, or blood samples is determined to be more likely to have a higher severity of PH. In the examination of the severity of PH, the level of indole compound concentrations in feces, gastrointestinal contents, or blood samples can be determined by dividing PH patients according to severity as a reference value in advance, obtaining the mean value or median value of the indole compound concentrations in feces, gastrointestinal contents, or blood samples, or the cutoff value for distinguishing between different severity levels, and comparing the measured value with the reference value.

Since the PH examination method (2) of the present disclosure can be performed as an examination for assisting diagnosis of PH, for a subject who is determined to have a high likelihood of having PH or having a likelihood of a high severity of PH by the PH examination method (2) of the present disclosure, it is desirable to perform further examinations such as catheterization, echocardiography, electrocardiography, and chest X-ray examination also for a definitive diagnosis.

In addition, in one aspect of the present disclosure, an examination kit used for performing the PH examination method (2) is provided. The examination kit may contain a reagent for measuring indole compounds. In addition, the examination kit may further contain an instrument for collecting feces or gastrointestinal contents, a reagent for extracting indole compounds from feces or gastrointestinal contents, and the like as necessary.

### 5. Method for Examining Presence or Absence of PH or Severity of PH Using AHR Activity in Feces or Gastrointestinal Contents as Indicator (PH Examination Method (3))

The present inventors have found that the indole compounds that are tryptophan metabolites are AHR ligands, and the AHR activity in feces and gastrointestinal contents is high due to the high concentration of the indole compounds in the intestinal tract of PH patients. Therefore, in one embodiment of the present disclosure, there is provided a method (hereinafter described as "PH examination method (3)") for examining the presence or absence of PH or the severity of PH, the examination method including a step of measuring the AHR activity in feces or gastrointestinal contents collected from a subject. Hereinafter, the PH examination method (3) of the present disclosure will be described in detail.

In the PH examination method (3) of the present disclosure, the subject, feces, and gastrointestinal contents are the same as those in the PH examination method (1).

In the PH examination method (3) of the present disclosure, AHR activity in feces or gastrointestinal contents may be determined by measuring transcriptional activity of AHR. The transcriptional activity of AHR can be measured by the following method. A reporter gene assay cell line is used, into which a plasmid vector has been introduced that contains a reporter gene linked downstream of a xenobiotic response element (XRE). Examples of the reporter gene include a luciferase gene. The reporter gene assay cell line is cultured in the presence of a sample isolated and prepared from a subject, and then the expression level of the reporter gene is measured. The expression level of the reporter gene is proportional to the activity (transcriptional activity) of AHR. Therefore, the activity of AHR in the sample can be evaluated by the expression level of the reporter gene. When a luciferase gene is used as the reporter gene, the amount of luminescence due to a catalytic reaction of luciferase generated by expression of the luciferase gene is measured by a luminometer, whereby the expression level of the luciferase gene can be measured. The reporter gene assay can be performed using commercially available reagents (including assay cell lines). As reagents for performing the AHR luciferase reporter assay, IB06001 from INDIGO Biosciences and the like are commercially available.

When the presence or absence of PH is examined in the PH examination method (3) of the present disclosure, a subject having high AHR activity in feces or gastrointestinal contents is determined to have a high likelihood of having PH. In the examination of the presence or absence of PH, the level of AHR activity in feces or gastrointestinal contents can be determined by obtaining in advance, as a reference value, the mean value or median value of the AHR activity in feces or gastrointestinal contents in healthy volunteers and/or PH patients, or the cutoff value for distinguishing between the healthy volunteers and the PH patients, and comparing the measured value with the reference value. For example, when the AHR activity in feces or gastrointestinal contents of a subject is higher than the mean value or median value of the AHR activity in feces or gastrointestinal contents of healthy volunteers, it can be determined that the subject is highly likely to suffer from PH. In addition, when the AHR activity in feces or gastrointestinal contents in a subject is equal to or higher than the mean value or median value of AHR activity in feces or gastrointestinal contents of PH patients, it can be determined that the subject is highly likely to suffer from PH.

In addition, when the severity of PH is examined in the PH examination method (3) of the present disclosure, a subject having a higher AHR activity in feces or gastrointestinal contents is determined to be more likely to have a higher severity of PH. In the examination of the severity of PH, the level of the AHR activity in feces or gastrointestinal contents can be determined by dividing PH patients according to severity as a reference value in advance, obtaining the mean value or median value of the AHR activity in feces or gastrointestinal contents, or the cutoff value for distinguishing between different severity levels, and comparing the measured value with the reference value.

Since the PH examination method (3) of the present disclosure can be performed as an examination for assisting diagnosis of PH, for a subject who is determined to have a high likelihood of having PH or having a likelihood of a high severity of PH by the PH examination method (3) of the present disclosure, it is desirable to perform further examinations such as catheterization, echocardiography, electrocardiography, and chest X-ray examination also for a definitive diagnosis.

In addition, in one aspect of the present disclosure, an examination kit used for performing the PH examination method (3) is provided. The examination kit may contain reagents (for example, reagents for performing AHR luciferase reporter assays) for measuring AHR activity. In addition, the examination kit may further contain an instrument for collecting feces or gastrointestinal contents, a reagent for extracting indole compounds from feces or gastrointestinal contents, and the like as necessary.

### 6. Method for Examining Presence or Absence of PH or Severity of PH Using ACE2 Concentrations in Feces, Gastrointestinal Contents, or Blood Samples as Indicator (PH Examination Method (4))

The present inventors have found that, in the intestinal tract of PH patients, the ACE2 expression level is decreased due to high concentrations of indole compounds (AHR ligands) that are tryptophan metabolites, and the ACE2 concentrations in feces, gastrointestinal contents, and blood samples are low. Therefore, in one embodiment of the present disclosure, there is provided a method (hereinafter described as "PH examination method (4)") for examining the presence or absence of PH or the severity of PH, the examination method including a step of measuring the ACE2 concentrations in feces, gastrointestinal contents, or blood samples collected from a subject. Hereinafter, the PH examination method (4) of the present disclosure will be described in detail.

In the PH examination method (4) of the present disclosure, the subject, feces, gastrointestinal contents, and blood samples are the same as those in the PH examination method (1).

The measurement of the ACE2 concentration in the gut microbiota or blood samples can be performed by a known ACE2 measurement method such as enzyme immunoassay (ELISA), immunoprecipitation method, Western blotting, or LC-MS/MS MRM. In addition, the ACE2 concentrations in blood samples may be measured by measuring soluble ACE2.

When the presence or absence of PH is examined in the PH examination method (4) of the present disclosure, a subject having low ACE2 concentrations in feces, gastrointestinal contents, or blood samples is determined to have a high likelihood of having PH. In the examination of the presence or absence of PH, the level of ACE2 concentrations in feces, gastrointestinal contents, or blood samples can be determined by obtaining in advance, as a reference value, the mean value or median value of the ACE2 concentrations in feces, gastrointestinal contents, or blood samples in healthy volunteers and/or PH patients, or the cutoff value for distinguishing between the healthy volunteers and the PH patients, and comparing the measured value with the reference value. For example, when the ACE2 concentrations in feces, gastrointestinal contents, or blood samples in a subject are lower than the mean value or median value of the ACE2 concentrations in feces, gastrointestinal contents, or blood samples in healthy volunteers, it can be determined that the subject is likely to suffer from PH. In addition, when the ACE2 concentrations in feces, gastrointestinal contents, or blood samples in a subject are equal to or lower than the mean value or median value of the ACE2 concentrations in feces, gastrointestinal contents, or blood samples in PH patients, it can be determined that the subject is likely to suffer from PH.

In addition, when the severity of PH is examined in the PH examination method (4) of the present disclosure, a subject having lower ACE2 concentrations in feces, gastrointestinal contents, or blood samples is determined to be more likely to have a higher severity of PH. In the examination of the severity of PH, the level of ACE2 concentrations in feces, gastrointestinal contents, or blood samples can be determined by dividing PH patients according to severity as a reference value in advance, obtaining the mean value or median value of the ACE2 concentrations in feces, gastrointestinal contents, or blood samples, or the cutoff value for distinguishing between different severity levels, and comparing the measured value with the reference value.

Since the PH examination method (4) of the present disclosure can be performed as an examination for assisting diagnosis of PH, for a subject who is determined to have a high likelihood of having PH by the PH examination method (4) of the present disclosure, it is desirable to perform further examinations such as catheterization, echocardiography, electrocardiography, and chest X-ray examination.

In addition, in one aspect of the present disclosure, an examination kit used for performing the PH examination method (4) is provided. The examination kit may include a reagent (for example, an anti-ACE2 antibody or the like) for measuring ACE2. In addition, the examination kit may further contain an instrument for collecting the gut microbiota, a reagent for extracting ACE2 from feces or gastrointestinal contents, and the like as necessary.

### 7. Prophylactic or Therapeutic Agent for PH by Mechanism of Increase in ACE2 Expression Level in Intestinal Tract (2)

The present inventors have found that, in the intestinal tract of PH patients, the ACE2 expression level is decreased due to high concentrations of indole compounds (AHR ligands) that are tryptophan metabolites, and that this leads to a decrease in a function of B0AT1, thereby forming the pathology of PH. Therefore, in one embodiment of the present disclosure, there is provided a prophylactic or therapeutic agent for PH (hereinafter referred to as "PH prophylactic or therapeutic agent (2)") containing a substance that increases the expression level of ACE2. Hereinafter, the PH prophylactic or therapeutic agent (2) of the present disclosure will be described in detail.

### [Active Ingredients]

The PH prophylactic or therapeutic agent (2) of the present disclosure contains a substance that increases the expression level of ACE2 as an active ingredient. In the PH prophylactic or therapeutic agent (2) of the present disclosure, PH can be prevented or treated by increasing the expression level of ACE2 in the intestinal tract to normalize the function of B0AT1.

As a substance that increases an expression level of ACE2, the substance is not particularly limited as long as the substance can promote expression of ACE2 and is pharmaceutically acceptable, and examples thereof include bile acids (such as ursodeoxycholic acid), a mineralocorticoid receptor antagonist, 17β-estradiol, atorvastatin, diminazene diaceturate, fibroblast growth factor 21 (FGF21), and polyinosinic acid-polycytidylic acid potassium salt (Poly(I:C)).

In the PH prophylactic or therapeutic agent (2) of the present disclosure, the substance that increases the expression level of ACE2 may be used alone, or may be used in combination with two or more thereof.

### [Formulation]

The PH prophylactic or therapeutic agent (2) of the present disclosure is provided by being prepared into a desired dosage form by blending the active ingredient with a pharmaceutically acceptable carrier, additive, or the like. Examples of pharmaceutically acceptable carriers or additives include sterile water, physiological saline, stabilizers, excipients, antioxidants, buffers, preservatives, surfactants, chelating agents, and binders.

Examples of the dosage form of the PH prophylactic or therapeutic agent (2) of the present disclosure include capsules, tablets, pills, sachets, liquids, powders, granules, fine granules, film-coated agents, pellets, troches, sublingual tablets, chewable tablets, buccal tablets, pastes, syrups, suspensions, elixirs, or emulsions. The content of the active ingredient in the PH prophylactic or therapeutic agent (2) of the present disclosure may be appropriately set according to the dose, dosage form, and the like.

### [Dosage and Administration]

In the PH prophylactic or therapeutic agent (2) of the present disclosure, the clinical classification of PH to be prevented or treated is not particularly limited, and the PH may be classified into any one of Group 1, Group 1', Group 1", Group 2, Group 3, Group 4, and Group 5 of the Nice classification. As one embodiment of the PH prophylactic or therapeutic agent (2) of the present disclosure, the PH prophylactic or therapeutic agent (2) can be used for preventing or treating Group 1 (PAH) of the Nice classification.

In addition, in the PH prophylactic or therapeutic agent (2) of the present disclosure, the severity of PH to be treated is not particularly limited, and may be any of the severity of Class I to Class IV in the WHO Functional Classification for pulmonary hypertension.

In the PH prophylactic or therapeutic agent (2) of the present disclosure, an administration route is not particularly limited as long as the active ingredient can be transferred into the intestine, and examples thereof include oral administration and enteral administration, and preferably oral administration.

The dose of the PH prophylactic or therapeutic agent (2) of the present disclosure may be appropriately set according to the age of the subject to be administered, the degree of PH pathology, the type of the active ingredient to be used, and the like, and for example, the active ingredient may be administered at approximately 1 to 100 mg/kg per day for an adult, preferably approximately 1 to 20 mg/kg per day, in 1 to 4 divided doses per day, preferably in 2 to 3 divided doses per day.

### Examples

Hereinafter, the present disclosure will be described more specifically with reference to examples, but the present disclosure is not to be construed as being limited to these examples.

### 1. Test Materials and Methods

### 1-1. Clinical Studies

With the approval of the Ethics Committee, fecal samples and blood samples were collected from 89 PH patients and 82 healthy volunteers as subjects. The PH patients consisted of 40 cases of idiopathic/heritable pulmonary arterial hypertension, 19 cases of connective tissue disease-associated pulmonary arterial hypertension, 10 cases of pulmonary hypertension associated with portal hypertension, 8 cases of chronic thromboembolic pulmonary hypertension, 7 cases of pulmonary arterial hypertension associated with congenital shunt disease, and 5 other cases. The healthy volunteers were a group matching age and sex. Table 2 shows the background factors of PH patients and healthy volunteers. Feces were collected from subjects and taken to the laboratory as soon as possible at 4°C and stored at -80°C until analysis. Blood samples were centrifuged at 3500 rpm for 5 minutes to collect serum and stored at - 80°C until analysis.

**[Table 2]**

| | PH patient (n = 89) | Healthy volunteer (n = 82) | p value |
|---|---|---|---|
| Age (years, IQR) | 47(35-57) | 44(34-51) | 0.099 |
| Female (n, %) | 22(25) | 26(32) | 0.310 |
| BMI (kg/m²) | 22.5±5.3 | 21.6±2.7 | 0.157 |
| WHO-FC 1/2/3/4 | 5/36/45/3 | - | - |
| Mean pulmonary arterial pressure (mmHg) | 39.0±13.5 | - | - |
| Vascular resistance (Wood units) | 9.2±6.1 | - | - |
| Cardiac index (L/min/m²) | 2.6±0.7 | - | - |

| | | | |
|---|---|---|---|
| The statistical values are mean ± standard deviation. | | | |

### 1-2. Measurement of Short-Chain Fatty Acid Concentration in Feces

To measure organic acids in feces, 100 mg of feces was placed in a 2.0 mL tube together with zirconia beads and suspended in Milli-Q. The samples were heated at 85°C for 15 minutes and vortexed at 5 m/s for 45 seconds using a FastPrep 24 5G (MP Biomedicals, CA, USA). After centrifugation at 15,350 × g for 10 minutes, the supernatant was filtered through a 0.2 µm filter. The organic acid concentrations in the filtrate were measured by a post-column reaction using high-performance liquid chromatography (Prominence, SHIMADZU, Kyoto, Japan) equipped with a detector (CDD-10A, SHIMADZU, Kyoto, Japan), two columns arranged in tandem (Shim-pack SCR-102(H), 300 mm × 8 mm ID, SHIMADZU, Kyoto, Japan), and a guard column (Shim-pack SCR-102(H), 50 mm × 6 mm ID, SHIMADZU, Kyoto, Japan). In this measurement, a mobile phase (5 mM p-toluenesulfonic acid) and a reaction solution (5 mM p-toluenesulfonic acid, 100 µM EDTA, and 20 mM Bis-Tris) were used.

### 1-3. Animal Experiments

All experiments were performed in accordance with the guidelines of the Animal Care and Use Committee of the National Cerebral and Cardiovascular Center and were approved by the Institutional Review Board of the National Cerebral and Cardiovascular Center. Ahr knockout (Ahr -/-) rats (Masaki et al., PNAS, 2021, 118(11):e2023899118), Ace2 knockout rats (Ace2 -/y), and Slc6a19 knockout rats (Slc6a19 +/-, -/-) were prepared using CRISPR/Cas9 gene editing. All the rats were housed at 24 ± 1°C under a 12-hour light/12-hour dark cycle and were fed a standard mouse diet and water.

### 1-3-1. Gnotobiotic Experiment

Germ-free male F344 rats were purchased from Japan SLC, Inc. and housed in a germ-free isolator environment. Feces derived from healthy volunteers or feces derived from pulmonary hypertension patients were administered to 11-week-old rats, and monocrotaline (MCT) was subcutaneously administered to 15-week-old rats to prepare pulmonary hypertension model gnotobiotic rats. Specifically, first, feces derived from four healthy volunteers were mixed in an anaerobic chamber to prepare a healthy volunteer fecal dilution obtained by diluting with an anaerobic transport medium (containing 20 g of Lablemco powder, 1 g of L-cysteine, 0.45 g of KH₂PO₄, 0.9 g of NaCl, 0.45 g of (NH₄)₂SO₄, 0.045 g of CaCl₂, 0.045 g of MgSO₄, 400 mL of glycerol, and 600 mL of distilled water per 1000 mL), and feces derived from four patients were similarly mixed in an anaerobic chamber to prepare a patient fecal dilution obtained by diluting with the anaerobic transport medium. Subsequently, a healthy volunteer fecal dilution (final 10 fold dilution) or a patient fecal dilution (final 10 fold dilution) was orally administered to 11-week-old rats in separate isolators, respectively, to prepare fecal transplantation gnotobiotic rats reproducing the gut microbiota of both healthy volunteers and patients. At 4 weeks after fecal transplantation (15 weeks of age), monocrotaline (60 mg/kg) (Sigma-Aldrich) was subcutaneously injected, and thereafter the rats were housed in an isolator for 3 weeks to prepare **PH** model gnotobiotic rats induced by monocrotaline loading. Table 3 shows basic information **on PH** patients and healthy volunteers used for fecal transplantation.

**[Table 3]**

| | Age | Sex | Diseases |
|---|---|---|---|
| Healthy volunteer 1 | 32 | Male | - |
| Healthy volunteer 2 | 53 | Female | - |
| Healthy volunteer 3 | 49 | Female | - |
| Healthy volunteer 4 | 47 | Female | - |
| PH patient 1 | 21 | Male | Idiopathic pulmonary arterial hypertension |
| PH patient 2 | 47 | Female | Connective tissue disease-associated pulmonary arterial hypertension |
| PH patient 3 | 59 | Male | Idiopathic pulmonary arterial hypertension |
| PH patient 4 | 54 | Female | Connective tissue disease-associated pulmonary arterial hypertension |

### (Gnotobiotic Experiment in Which AHR Inhibitor Was Administered)

Feces derived from patients were administered to 11-week-old germ-free rats, and monocrotaline was subcutaneously administered at 15 weeks of age to prepare pulmonary hypertension model gnotobiotic rats. From the day after monocrotaline administration, the vehicle group was administered corn oil, and the AHR inhibitor group was administered CH223191 (Selleck) at 8 mg/kg/day by daily gavage. On day 21 after monocrotaline administration, measurement of right ventricular systolic pressure and Fulton's index, and collection of feces, blood, and organs were performed.

### (Gnotobiotic Experiment Using Low Tryptophan Diet or High Tryptophan Diet)

Feces derived from patients were administered to 11-week-old germ-free rats, and at 13 weeks of age, the rats were divided into a low tryptophan diet group and a high tryptophan diet group and then housed. The low tryptophan diet group was fed a diet containing 0.1 w/w% tryptophan, while the high tryptophan diet group was fed a diet containing 1.25 w/w% tryptophan, and monocrotaline was subcutaneously administered at 15 weeks of age to induce pulmonary hypertension. On day 21 after monocrotaline administration, measurement of right ventricular systolic pressure and Fulton's index, and collection of feces, blood, and organs were performed.

### (Gnotobiotic Experiment in Which Single Bacterium or Two Bacteria Were Transplanted)

Ruminococcus gnavus (JCM6515) and Streptococcus pasteurianus (isolated from rat feces transplanted with patient feces) were cultured in trypticase soy broth under anaerobic conditions at 37°C. 11-week-old germ-free rats were inoculated with Ruminococcus gnavus and Streptococcus pasteurianus, either individually or in combination. Monocrotaline was subcutaneously administered at 15 weeks of age to induce pulmonary hypertension. On day 21 after monocrotaline administration, measurement of right ventricular systolic pressure and Fulton's index, and collection of feces, blood, and organs were performed.

### (Gnotobiotic Experiment with AST-120 Administered)

Feces derived from patients were administered to 1-week-old germ-free rats, and monocrotaline was subcutaneously administered at 15 weeks of age to prepare pulmonary hypertension model gnotobiotic rats. From the day after monocrotaline administration, the vehicle group was fed a standard diet (CRF-1 powder diet), and the AST-120 group was fed a CRF-1 powder diet containing 8 w/w% AST-120. On day 21 after monocrotaline administration, measurement of right ventricular systolic pressure and Fulton's index, and collection of feces and organs were performed.

### 1-3-2. Experiments in Specific Pathogen Free Environment

The experiment in the SPF environment was performed using Sprague-Dawley (SD) rats purchased from Charles River Laboratories Japan. Male rats at 6 to 8 weeks of age were used.

### 1-3-3. Experiment Using Regnase-1 CD11c-Cre Mice

Regnase-1 CD11c-Cre (Reg-1 CKO) mice, which were generously provided by Professor Osamu Takeuchi of the Graduate School of Medicine, Kyoto University (Yaku et al., 2022, Circulation, 146(13):1006-1022), were used in this study.

### 1-3-4. Experiments in Which Tryptamine Was Administered

A vehicle group of 13-week-old germ-free F344 rats was fed a standard diet (CRF-1 powder diet), a tryptamine group was fed a CRF-1 powder diet containing 1.25 w/w% tryptamine, and at 15 weeks of age, 60 mg/kg of a monocrotaline was subcutaneously administered to induce pulmonary hypertension. On day 21 after monocrotaline administration, measurement of right ventricular systolic pressure and Fulton's index, and collection of feces, blood, and organs were performed.

### 1-3-5. Experiments with AST-120 Administered

### (SD Rats; Measurement of Tryptophan Metabolites)

6-week-old male SD rats were divided into a vehicle group and an AST-120 group. A vehicle group was fed a standard diet (CRF-1 powder diet), and an AST-120 group was fed a CRF-1 powder diet containing 8 w/w% AST-120, feces on day 7 were collected, and tryptophan metabolites (indole compounds) in feces were measured using

### LC-MS.

### (Monocrotaline-Loaded SD Rats; Measurement of Right Ventricular Systolic Pressure)

6-week-old male SD rats housed in the SPF environment were divided into a vehicle group and an AST-120 group. Both groups of rats were subcutaneously administered 60 mg/kg of monocrotaline at 6 weeks of age to induce pulmonary hypertension, a vehicle group was fed a standard diet (CRF-1 powder diet), and an AST-120 group was fed a CRF-1 powder diet containing 8 w/w% AST-120, and both groups were then housed for 3 weeks. The right ventricular systolic pressure was measured 3 weeks after monocrotaline administration.

### (Hypoxia-Loaded SD Rats; Measurement of Right Ventricular Systolic Pressure)

6-week-old male SD rats housed in the SPF environment were divided into a vehicle group and an AST-120 group. Both groups of rats were continuously housed for 4 weeks in a chamber maintained under 10% oxygen conditions. During a housing period under 10% oxygen conditions, the vehicle group was fed a standard diet (CRF-1 powder diet), and the AST-120 group was fed a CRF-1 powder diet containing 8 w/w% AST-120. After 4 weeks of housing under 10% oxygen conditions, the right ventricular systolic pressure was measured.

### (Regnase-1 CD11c-Cre Mice)

4-week-old Regnase-1 CD11c-Cre (Regnase-1 KO) mice were divided into a vehicle group and an AST-120 group. A vehicle group was fed a standard diet (CRF-1 powder diet), and an AST-120 group was fed a CRF-1 powder diet containing 8 w/w% AST-120, and after 4 weeks, the right ventricular systolic pressure and the Fulton's index were measured.

### 1-4. Measurement of Right Ventricular Systolic Pressure

The rats were sedated and analgesized by inhalation anesthesia with Isoflurane (Pfizer). The body temperature of the rats during the procedure was maintained between 37°C and 38°C on a thermostatically controlled heat pad coupled with a rectal temperature monitor. The tracheotomy was performed, and ventilation was carried out using a rat ventilator (Harvard Apparatus) at a tidal volume of 10 µL/g and 70 breaths/min. A polyethylene tube was inserted into the right external jugular vein and advanced to a right ventricle to measure the right ventricular pressure (RVP). The RVP signal was detected by a pressure transducer (MLT0670; ADInstruments), the signal was relayed by a pressure amplifier (ML117; ADInstruments), continuously sampled by a PowerLab system (ADInstruments, Colorado Springs, CO), and recorded on a computer using Chart software (ADInstruments). The heart rate was calculated based on the peak in the right ventricular systole.

The right ventricular systolic pressure in mice was measured under the same conditions as in the case of the rat except that (1) a ventilator was set to 160 to 170 breaths/min, and (2) a 24G needle into which a polyethylene tube catheter had been inserted was inserted into a right ventricle from below a diaphragm to measure the RVP.

### 1-5. Measurement of Fulton's Index

After measurement of right ventricular systolic pressure, rats were euthanized with an overdose of anesthetic, and the hearts were excised. After removing the atria, the right ventricle was isolated from the left ventricle and the septum. After removing water, the weight of the right ventricle and the weight of the left ventricle + septum were measured, and the weight ratio of both (right ventricle/left ventricle weight ratio) was calculated as a Fulton's index to evaluate hypertrophy of the right ventricle.

### 1-6. RNA-seq Analysis

The quality of RNA and library preparation was assured by TapeStation (Agilent). For RNA-sequencing analysis of rat lungs, 100 ng of total RNA was used for ribosomal RNA (rRNA) depletion, and thereafter library preparation was performed using a TruSeq Stranded mRNA Sample Preparation Kit (Illumina). For RNA-sequencing analysis of human or rat peripheral blood mononuclear cells (PBMCs), 100 pg of total RNA was used, and library preparation was performed using the SMART-seq v4 Ultra Low Input RNA Kit (Takara Clontech). For each sample, more than 25 million reads were obtained, with 75 bp paired-end reads. Quality control of the sequencing data was performed using FastQC. Each read subjected to trimming and filtering was aligned, for rats, to a rat genome Version rn6 reference genome using rat genome (Rn6), and for humans, to a human genome (hg38) reference genome using Hisat2. The gene group specifically expressed in lung tissue was defined as a gene group in which the expression level change of 2 times or more or 1/2 or less was observed and a False Discovery Rate (FDR) < 0.05 was satisfied. The gene group specifically expressed in PBMC was defined as a gene group that exhibited an expression change of 1.5 times or more in humans and 2 times or more in rats, and satisfied P < 0.05.

### 1-7. Metagenomic Analysis

### (16S Analysis)

DNA was extracted from feces using NecleoSpin DNA stool (Macherey-Nagel). Using 16S Metagenomic Sequencing Library Preparation (Illumina) and a primer set targeting V1-V2 of 16S ribosomal RNA (27Fmod: 5'-AGRGTTTGATCMTGGCTCAG-3' (SEQ ID NO: 1) and 338R: 5'-TGCTGCCTCCCGTAGGAGT-3' (SEQ ID NO: 2)), a library was prepared according to a product protocol. The 251 base amplicon was sequenced using the MiSeq System (Illumina). The obtained paired-end reads were merged using PEAR (sco.hits.org/exelixis/web/software/pear/) and trimmed by BBtrim (bbmap.sourceforge.net). Furthermore, 20,000 reads were randomly extracted per sample by random_sequence_sample. pl(ualberta.ca/~stothard/software.html). The treated sequences were clustered into operational taxonomic units (OTUs) from 99% homology based on UCLUST version 1.2.33q. Each OTU was taxonomically classified by referencing the Silva v138 database, and bioinformatic microbiota analysis was performed using QIIME version 2.

### (Shotgun Analysis)

Whole-genome sequencing was performed on a DNBSEQ-G400 system (MGI Tech), and 150-base paired-end reads were generated. The main steps of the quality check (QC) process are (i) trimming of low quality bases, (ii) removal of duplicate reads, (iii) identification and masking of human reads. Duplicate reads were marked using PRINSEQ-lite (version 0.20.4). Raw reads were trimmed using Trimomatic (version 0.39) to clip Illumina adapters and to cut low quality bases at both ends. Reads having a length of less than 60 bp after trimming were discarded. Next, deduplication was performed by retaining only the longest read among duplicates of identical sequences. As the final stage of QC, quality-filtered reads were aligned to the human reference genome (hg38) using bowtie2 (version 2.3.5) and BMTagger (version 3.101). In both tools, only reads for which paired-end alignment failed were retained. For phylogenetic annotation and quantification of abundance, filtered paired-end reads were aligned to reference genome datasets using Bowtie2. For multi-mapped reads, only the best alignment was selected based on the alignment score. The number of reads mapped to each genome was divided by the length of the genome. The values of each genome were summed for each sample to calculate the relative abundances of species-level clades.

Filtered paired-end reads were assembled de novo into contigs using MEGAHIT (version 1.2.9). Open reading frames (ORFs) on the contigs were predicted using MetaGeneMark (version 3.38). Next, annotation of the ORF catalog was performed using Kyoto Encyclopedia of Genes and Genomes (KEGG) protein database (https://www.kegg.jp). Annotation was performed using a database of KEGG genes of prokaryotes and MGENES, which is a database of KEGG genes of a metagenome sample. Putative amino acid sequences translated from the ORF catalog and the KEGG protein database were aligned using DIAMOND with BLASTP (version v0.9.32.133). To quantify ORF abundance, filtered paired-end reads were mapped to the assembled contig using bowtie2 with default parameters. To avoid gene size bias, the abundance of ORF was defined as the depth of the region of each ORF in the ORF catalog according to the mapping results. After sample QC, QC and normalization of bacterial abundance data were performed. Next, normalization of ortholog abundance data of microbial genes was also performed.

### 1-8. Luciferase Assay

The AHR luciferase reporter assay was performed using IB06001 of INDIGO Biosciences according to instructions, except for stimulation conditions. For stimulation, a fecal extract with serum or PBS was added to a medium described in instructions such that a final concentration was 10 w/v%.

### 1-9. LC-MS Analysis

Table 4 shows standard substances of tryptophan and indole compounds measured by LC-MS analysis.

**[Table 4]**

| Compound name | Abbreviations | CAS | Mfg. |
|---|---|---|---|
| L-tryptophan | Trp | 73-22-3 | Tokyo Chemical Industry |
| Indole-3-aldehyde (indole-3-carboxaldehyde) | IAld | 487-89-8 | Tokyo Chemical Industry |
| Indole-3-pyruvic acid | IPyA | 392-12-1 | Sigma Aldrich |
| Indole-3-ethanol (tryptophol) | IEt | 526-55-6 | Tokyo Chemical Industry |
| Indole-3-acetic acid | IAA | 87-51-4 | Tokyo Chemical Industry |
| Tryptamine | TryptA | 61-54-1 | Tokyo Chemical Industry |
| Indole-3-propionic acid | IPA | 830-96-6 | Tokyo Chemical Industry |
| Indole-3-acrylic acid | IAcr | 1204-06-4 | Tokyo Chemical Industry |
| Indole-3-lactic acid | ILA | 1821-52-9 | MedChemExpress |

### 1-10. Preparation of Measurement Sample

Serum was centrifuged after addition of four volumes of methanol and vortexing, and the obtained supernatant was used for analysis. Feces were homogenized by bead disruption after addition of four volumes of 0.1% formic acid aqueous solution. The supernatant after centrifugation was mixed with an equal amount of methanol. The sample mixed with methanol was further centrifuged, and the obtained supernatant was filtered and used for analysis. In the measurement of IPyA in serum and feces, when adding methanol, three volumes of 0.25 mol/L 2-aminoethanethiol (pH 8.0) relative to the supernatant were added, and after reacting at room temperature for 1 hour, a supernatant obtained by centrifugation was measured as a TAZ form.

### 1-11. LC-MS Analysis

Ultra-high-performance liquid chromatography (UHPLC) isolation was performed using a Nexera ultra-high-performance liquid chromatograph (manufactured by Shimadzu), and as a column, a Shim-pack Scepter C18 (inner diameter 2.1 mm, length 100 mm, particle diameter 3 µm) manufactured by Shimadzu GLC was used. A mobile phase A was 0.1% formic acid aqueous solution, and a mobile phase B was 0.1% formic acid acetonitrile. For mass spectrometry, LCMS-8060NX or 8060 triple quadrupole mass spectrometer (Shimadzu Corporation) was used. The MRM transition optimization was performed with each standard solution. Initial data of metabolites were analyzed with LabSolutions LCMS (Shimadzu).

Multicomponent simultaneous LC-MS analysis was performed by relative comparison based on an area value using a method package (cell culture profiling, primary metabolites) of Shimadzu Corporation. As a column, Supelco Discovery HS F5-3 with an inner diameter of 2.1 mm, a length of 150 mm, and a particle diameter of 3 µm was used. A mobile phase A was 0.1% formic acid aqueous solution, and a mobile phase B was 0.1% formic acid acetonitrile. For mass spectrometry, LCMS-8060NX triple quadrupole mass spectrometer (Shimadzu Corporation) was used.

### 1-12. Single-Cell RNA-seq Analysis

Immune cells were isolated from surgically collected lungs. Specifically, the lung tissue was cut with scissors in a digestion solution obtained by adding collagenase (Sigma-Aldrich), DNase I (Roche), and Dispase (Thermo Fisher Scientific) to Hanks' balanced salt solution (HBSS), and then mechanically disrupted using gentleMACS (Miltenyi Biotec). The obtained cells were subjected to Percoll density gradient centrifugation for the enrichment of immune cells, and the fraction containing immune cells was collected. Immune cells were isolated using FACS Aria Fusion (BD Biosciences). Dead cells were removed with 7-Amino-Actinomycin D (7-AAD) staining. Sorted cells were loaded into Chromium Next GEM Chip G (10x Genomics) on the Chromium Controller (10x Genomics) and barcoding and cDNA synthesis were performed. cDNA amplification and library construction were performed using the Chromium Next GEM Single Cell 3' GEM, Library & Gel Bead Kit v3.1 or the Chromium Next GEM Single Cell 3' Kit v3.1 for 3' profiling (10x Genomics), according to the manufacturer's instructions. The library was sequenced with DNBSEQ-G400 (MGI Tech). Droplet libraries were processed using Cell Ranger 5.0.0 (10X Genomics). Sequence reads were aligned to the GRCh38 human reference genome using STAR (v2.7.2a)50. Analysis was performed using the filtered expression matrix generated using Cell Ranger count. In each sample, cells with a Unique Molecular Identifier (UMI) below the 1st percentile or above the 99th percentile were excluded. Further, cells having fewer than 200 expressed genes and cells in which reads from mitochondrial genes or hemoglobin genes accounted for 10% or more were also excluded. Uniform manifold approximation and projection (UMAP) projection, cell type annotation, and expression difference analysis were performed using BBrowser X (BioTuring).

### 1-13. Experiment with CACO-2 Cells (in vitro)

The human CACO-2 colorectal adenocarcinoma cell line obtained from ATCC was used. The medium used was a minimum essential medium (MEM; Nacalai Tesque) supplemented with 20% fetal bovine serum (Sigma-Aldrich), 1% non-essential amino acids (Nacalai Tesque), 100 U/ml penicillin, and 100 µg/mL streptomycin (Nacalai Tesque). CACO-2 cells were seeded in 12-well plates and cultured at 37°C in a 5% CO₂ humidified incubator. The medium was replaced twice a week, and experiments were performed 21 days after seeding. In the experiment, tryptamine or BAY-218 (AChemBlock) was added to the medium at a predetermined concentration, and after 72 hours, cells were collected, and the expression level of the ACE2 protein was measured.

### 1-14. Tritium-labeled Tryptophan Uptake Experiment (Ex-vivo)

As tritium-labeled tryptophan, ³H-tryptophan, L-[5-³H] (American Radiolabeled Chemicals) was used. After washing the small intestine of rats with cold physiological saline, the everted intestinal ring was prepared using a 1 mL pipette.
The uptake experiment was performed by adding 0.1 µCi tritium-labeled tryptophan to HBSS (Hanks' Balanced Salt Solution, containing Ca2+ and Mg2+) containing cOmplete (Roche), and incubating at room temperature for 5 minutes. Na-free HBSS was used in which NaCl was replaced with choline-Cl and Na₂HPO₄ was replaced with K₂HPO₄. After washing the intestine with Na-free HBSS, it was dissolved in a liquid scintillation (pico-fluor plus, PerkinElmer), and radioactivity was counted using a liquid scintillation counter (Tri-Carb 4910TR, PerkinElmer). The ratio of the radioactivity taken up under Na-free HBSS to the radioactivity taken up under Na⁺ HBSS was calculated to evaluate a Na-dependent tryptophan uptake amount.

### 1-15. Western Blotting

Proteins were extracted from a Caco-2 monolayer or a rat-derived small intestinal epithelial cell layer in the radioimmunoprecipitation assay buffer (50 mM Tris pH 7.4, 150 mM sodium chloride, 1% NP40, 0.5% sodium deoxycholate, 0.1% SDS, 1 mM EDTA, cOmplete protease inhibitor mixture (Roche)). After centrifugation at 20,000 × g at 4°C for 15 minutes, the protein was quantified using a BCA protein assay kit (Thermo Fisher Scientific). Equal amounts of protein were loaded onto SuperSep Ace 5-20 (Fujifilm) and transferred to 0.2 µm polyvinylidene fluoride plus membranes. After blocking with 5% skim milk (Nacalai Tesque) at room temperature for 1 hour, the membranes were incubated with the following primary antibodies overnight at 4°C. As primary antibodies, an anti-ACE2 antibody (Novus Biologicals) and an anti-β-actin antibody (Cell Signaling Technology) were used. After washing, the membranes were incubated with a horseradish peroxidase (HRP)-labeled secondary antibody (Cell Signaling Technology) at room temperature for 1 hour, and visualized with ECL-plus (Thermo Fisher Scientific) and an imaging system (Cytiva). Protein expression was normalized to expression of β-actin, and densitometry of banding of the Western blot was analyzed using ImageJ.

### 1-16. Bacterial Culture (in Vitro)

Ruminococcus gnavus (JCM6515), Streptococcus salivarium (JCM5707), Streptococcus pasteurianus (isolated from feces of patient feces-transplanted gnotobiotic rats at the National Cerebral and Cardiovascular Center), Streptococcus mutans (JCM5705), and Rothia aeria (JCM11412) were cultured in Trypticase soy medium under anaerobic conditions at 37°C, and indole compounds derived from tryptophan were measured in a culture supernatant using LC-MS. Ruminococcus gnavus was cultured for 24 hours in a Trypticase soy medium and a Trypticase soy medium containing 0.5% mucin (Sigma-Aldrich), and turbidity (number of bacteria) was measured using a MicroScan Turbidity Meter (Beckman Coulter).

### 1-17. qPCR

Lung tissues from rats or humans, or lysates of PBMCs or cultured cells, were lysed using TRIzol (Invitrogen), and a total RNA was extracted using a PureLink RNA Mini Kit (Invitrogen) and PureLink DNase (Invitrogen). Quantitative real-time RT-PCR was performed using either the QuantiFast SYBR Green RT-PCR Kit (Qiagen) or a combination of the PrimeScript RT Reagent Kit (Takara Bio) and FastStart Essential DNA Green Master (Roche). In a case where the former was used, 80 ng of a total RNA was reverse-transcribed at 50°C for 10 minutes and then denatured at 95°C for 5 minutes, followed by performing a qRT-PCR reaction at 95°C for 10 seconds and 60°C for 30 seconds for 45 cycles. Fluorescence data were collected and analyzed using a LightCycler 96 (Roche). The genes used and primers thereof are as follows.
Gapdh:
   FW: AAAGGGTCATCATCTCCGCC (SEQ ID NO. 3)
   RV: AGTGATGGCATGGACTGTGG (SEQ ID NO. 4)
Mucin2:
   FW: ACCACCATTACCACCACCTCAG (SEQ ID NO. 5)
   RV: CGATCACCACCATTGCCACTG (SEQ ID NO. 6)

### 1-18. Measurement of Adsorption Capacity of Adsorbent Carbon

L-valine, L-glutamic acid, and 3-aminoisobutyric acid were each dissolved in PBS(-) to be 20 mg/mL to prepare stock solutions. Further, L-tryptophan, tryptamine, indole-3-aldehyde, and indole-3-acetic acid were each dissolved in dimethyl sulfoxide to be 100 mg/mL to prepare stock solutions. Each of the obtained stock solutions was mixed and diluted with PBS(-) to prepare a compound mixture in which each compound was contained at a concentration of 0.1 mg/mL.

20 mg of each carbonaceous adsorbent of AST-120 (CAS: 90597-58-3, Kureha Corporation), medicinal carbon (CAS: 16291-96-6, Nichi-Iko Pharmaceutical), and granulated activated carbon (CAS: 7440-44-0, UES Co., Ltd.) was placed in a 15 mL tube, and further the compound mixture was added to make the total amount of the carbonaceous adsorbent and the compound mixture 10 mL. Subsequently, after gently stirring at 37°C for half a day, the carbonaceous adsorbent was removed by filtration, and a filtrate was collected. The obtained filtrate was subjected to LC-MS analysis, and the concentration of each compound was quantified. Further, as a control, the same procedures as described above were performed except that the carbonaceous adsorbent was not added. The adsorption rate of the carbonaceous adsorbent for each compound was calculated according to the following equation. $Adsorption rate = \left\{\left(A-B\right)/A\right\} \times 100$
A: Compound concentration of filtrate of control
B: Compound concentration in filtrate with carbonaceous adsorbent added

### 1-19. Statistics

All data were expressed as a mean value ± a standard error or a standard deviation. A test for significant differences among multiple groups was performed by one-way ANOVA, followed by a post-hoc test. The test between the two groups was analyzed by Student's t-test or Mann-whitney test. The event-free survival curve was derived using a Kaplan-Meier method and compared using a log-rank test. Network analysis was performed using Spearman's rank correlation. A p value of less than 0.05 was considered as statistically significant.

### 2. Contents Reported by Inventor (NPL 6)

### 2-1. In PAH Patients, AHR Activity in Serum Is Increased, and AHR Activity in Serum Reflects Severity of PAH

It has been revealed that the AHR agonist activity in serum (AHR-Luc activity) of PAH patients and healthy volunteers (HV) is significantly higher in PAH patients than in HV (Fig. 2A). It has also been found that AHR-Luc activity in serum is significantly higher in more severe PAH (Class III and Class IV in the WHO Functional Classification for pulmonary hypertension) than in relatively mild PAH (Class I and Class II in the WHO Functional Classification for pulmonary hypertension) (Fig. 2B). Further, it has also been reported that, in the PAH patients having the high AHR-Luc activity, the likelihood of severe clinical events such as death, lung transplantation, and hospitalization for treatment of right heart failure occurring is significantly higher as compared with the PAH patients having low AHR-Luc activity (Fig. 2C). As described above, the present inventors have previously demonstrated that the activation of AHR plays an important role in onset of PAH, and that activation of AHR in serum can serve as an indicator of severity of PAH.

### 2-2. FICZ, Endogenous AHR Ligand, in Combination with Hypoxic Stimulation, Induces Severe Pulmonary Hypertension Accompanied by Intimal Lesions in Rats

6-formylindolo[3,2-b]carbazole (FICZ) is an endogenous potent AHR agonist. It has been reported that, when rats are subjected to the hypoxic load (10% O₂) for the first 3 weeks and FICZ is subcutaneously administered (FICZ/Hx/Nx rat model; Fig. 2A), a marked increase in right ventricular systolic pressure (Fig. 2B) and Fulton's index (Fig. 2C) is observed. Further, it has also been reported that, in a distal small artery of the FICZ/Hx/Nx rat model, not only medial thickening (Figs. 2D and 2E) but also appearance of an occlusive lesion due to neointimal proliferation (Figs. 2D, 2F, and 2G) were observed. Thus, the present inventors have already found that AHR activation is one factor causing onset and aggravation of PH.

### 2-3. In SuHx Rat Model, When AHR Is Knocked Out, PAH Pathology Is Markedly Suppressed

The SuHx rat model is a model in which a VEGF receptor antagonist SU5416 is administered, a hypoxic load is applied, and a normoxic load is applied to induce a PAH pathology. Using a CRISPR/Cas9 gene editing system, an AHR knockout SuHx rat model (Ahr-/- rat) was prepared, and regarding the results of observation of PAH pathology, the following report has been made. No increases in right ventricular systolic pressure (Fig. 3B) or the Fulton's index (Fig. 3C) were observed in Ahr-/- rats. The medial thickness index of the distal small artery was markedly decreased in Ahr-/- rats as compared with Ahr+/+ rats (Figs. 3D and 3E). In addition, no occlusive vessels were observed in Ahr-/- rats (Figs. 3D, 3F, and 3G). This indicates that the effect of inhibiting the AHR signal is effective not only for medial thickening (which is also observed in a mild pulmonary hypertension model such as in mice) but also for suppression of appearance of a neointimal lesion and a plexiform lesion observed in severe pulmonary hypertension in humans, suggesting a possibility that it is also effective for severe cases and treatment-resistant cases in which sufficient pathological improvement cannot be obtained with existing drugs. Thus, the present inventors have already found that inhibition of AHR is effective for suppression of onset of PAH.

### 3. Reference Test Results

### 3-1. In PH Patients, Dysbiosis of Gut Microbiota Characterized by Ectopic Colonization of Oral Bacteria is Observed

The gut microbiota of PH patients (PH, 89 cases) and healthy volunteers (HV, 82 cases) was analyzed. In the gut microbiota of the PH patients, Faith PD and Shannon index, which are indicators of α-diversity, were significantly decreased as compared with the healthy volunteers (Fig. 4A). In addition, in the gut microbiota of the PH patients, β-diversity (unweighted unifrac PCoA) was greatly changed as compared with healthy volunteers (Fig. 4B).

In addition, from the results of the volcano plot of the composition analysis (ANCOM) of the gut microbiota, it was found that in the PH patients, oral bacteria such as Streptococcus, Rothia, and Actinomyces increased in the intestine, and short-chain fatty acid (SCFA)-producing bacteria such as Subdolignulum, Ruminococcus, and Coprococcus decreased (Fig. 5A). In addition, when the oral resident bacteria score (OS) was obtained by scoring the number of species of oral resident bacteria colonizing in the intestine (core 14 species possessed by more than 90% of all individuals at a frequency of 1% or higher), the results showed that the oral resident bacteria score in the intestine of PH patients was significantly increased as compared with that of healthy volunteers (Fig. 5B). Furthermore, PH patients with a high oral resident bacteria score (OS > 5) had a higher PH functional class of WHO and were more severe (Fig. 5C) and had a lower event-free survival rate (Fig. 5D) than PH patients with a low oral resident bacteria score (OS ≤ 5).

In addition, in feces of PH patients, the concentrations of acetic acid, propionic acid, and butyric acid were significantly reduced as compared with the feces of healthy volunteers (Fig. 5E), suggesting that the intestinal environment deviated from an extremely healthy state.

From these results, it was confirmed that, in the gut microbiota of a PH patient, dysbiosis due to colonization of oral bacteria has occurred.

### 3-2. In Intestine of PH Patients, Ruminococcus Gnavus Coexists by Forming Networks with Multiple Oral Bacteria in Intestinal Tract, Which Is Associated with Poor Prognosis

From the results of the composition analysis of the gut microbiota of the PH patients, the relative frequency of Ruminococcus gnavus was high in the PH patients having a high oral resident bacteria score. Furthermore, when the network analysis was performed on bacteria correlated with Ruminococcus gnavus (Spearman's rank correlation coefficient > 0.2), it was found that, in the intestine, the Ruminococcus gnavus forms a network and coexists with Streptococcus, Veillonella, Rothia, and Fusobacterium (all core oral bacteria) (Fig. 6A). Further, it was also found that, in PH patients in whom Ruminococcus gnavus coexists with Streptococcus, Rothia, Fusobacterium, or Veillonella in the intestine, a prognosis becomes markedly poor (Log-rank test; Streptococcus P = 0.007, Rothia P = 0.004, Veillonella P = 0.023) (Fig. 6B). From these results, it has been revealed that, in PH patients, Ruminococcus gnavus coexists by forming networks with multiple oral bacteria in the intestinal tract, which is associated with poor prognosis.

### 3-3. Pulmonary Hypertension Model Rats Exhibit Dysbiosis of Gut Microbiota (WO 2021/167088 A).

There are three representative disease models for PH using rats (Fig. 7A). The dysbiosis of the gut microbiota was confirmed in these rat models.

6-week-old male SD rats were continuously housed in a hypoxic chamber at 10% oxygen for 3 weeks to prepare a pulmonary hypertension model rat by hypoxic load (Hx) (Fig. 7A). Monocrotaline was subcutaneously administered to 6-week-old male SD rats at 60 mg/kg, and the rats were housed for 3 weeks under the normoxic environment (normoxia) to prepare a pulmonary hypertension model rat by monocrotaline load (MCT) (Fig. 7A). After subcutaneously administering Sugen5416, which is a VEGFR2 inhibitor, to 6-week-old male SD rats at 20 mg/kg, the rats were continuously housed in a hypoxic chamber at 10% oxygen for 3 weeks and then housed for 2 weeks under the normoxic environment (normoxia) to prepare a pulmonary hypertension model rat by Sugen5416/hypoxic load (SuHx) (Fig. 7A).

Analysis of the gut microbiota of each pulmonary hypertension model rat revealed that β-diversity was changed in Hx, MCT, and SuHx model rats (Fig. 7B). However, a gut microbiota of rats is greatly different from a gut microbiota of humans, oral bacteria including Streptococcus are hardly detected in rats, bacteria belonging to Mulibaculaceae are commonly increased, and Lachnospiraceae is commonly decreased (Fig. 7C).

### 3-4. Administration of Antibiotic to Pulmonary Hypertension Model Rats Improves PH Pathology (WO 2021/167088 A)

Water containing ampicillin (1 g/L), neomycin (1 g/L), metronidazole (1 g/L), and vancomycin (1 g/L) (antibiotic cocktail water) or water was administered to each of Hx, MCT, and SuHx model rats, and a PH pathology (pulmonary arterial medial thickening, right ventricular systolic pressure, and right ventricle/left ventricle weight ratio) was evaluated. In the case of Hx, after allowing free drinking of antibiotic cocktail water or water during a housing period of 3 weeks in a hypoxic chamber at 10% oxygen, the PH pathology was evaluated. In the case of MCT, after allowing free drinking of antibiotic cocktail water during a housing period of 3 weeks under the normoxic environment (normoxia) after the monocrotaline administration, the PH pathology was evaluated. In SuHx, after allowing free drinking of antibiotic cocktail water or water during a housing period of 2 weeks under the normoxic environment (normoxia) after hypoxic load, the PH pathology was evaluated. Further, in a control, male SD rats in which PH was not induced were housed under the condition of allowing free drinking of water. As a result, in all PH model rats administered an antibiotic cocktail, significant decreases in pulmonary arterial medial thickening, right ventricular systolic pressure, and right ventricle/left ventricle weight ratio were observed as compared with the case in which water was administered (Figs. 8A to 8C).

### 3-5. In PH Model Rats, When Gut Microbiota of PH Patients Is Reconstituted, PH Pathology Is Promoted, and Inflammatory Signaling in Lung and Responses to Xenobiotics Are Enhanced

PH model gnotobiotic rats (PH patient feces-transplanted F344 rats or healthy volunteer feces-transplanted F344 rats) were prepared by administering a fecal dilution derived from PH patients or healthy volunteers to 11-week-old germ-free F344 rats, administering monocrotaline at 15 weeks of age, and further housing the rats for 3 weeks (Fig. 9A). Further, germ-free environment-housed F344 rats were prepared by administering monocrotaline at 15 weeks of age to 11-week-old germ-free F344 rats without administering a fecal dilution, and further housing the rats for 3 weeks. Further, SPF environment-housed F344 rats were prepared by housing 11-week-old germ-free F344 rats in an SPF environment of the National Cerebral and Cardiovascular Center without administering a fecal dilution, administering monocrotaline at 15 weeks of age, and further housing the rats for 3 weeks in the SPF environment.

When a composition of a gut microbiota of PH patient feces-transplanted F344 rats and healthy volunteer feces-transplanted F344 rats was analyzed, the composition of the gut microbiota was greatly different between them. Streptococcus, which is an oral bacterium, colonized extensively in the PH patient feces-transplanted F344 rats, and Faecalibacterium, which is a representative butyrate-producing bacterium, colonized extensively in the healthy volunteer feces-transplanted F344 rats (Fig. 9B).

Further, when right ventricular systolic pressure and right ventricle/left ventricle weight ratio of each rat were measured, in germ-free environment-housed F344 rats, both the right ventricular systolic pressure and the right ventricle/left ventricle weight ratio were decreased as compared with SPF environment-housed F344 rats, and monocrotaline-induced PH was significantly suppressed (Fig. 9C). Further, healthy volunteer feces-transplanted F344 rats showed right ventricular systolic pressure and right ventricle/left ventricle weight ratio comparable to those of germ-free environment-housed F344 rats, and monocrotaline-induced PH was significantly suppressed (Fig. 9C). Furthermore, in patient feces-transplanted F344 rats, the right ventricular systolic pressure was significantly increased as compared with healthy volunteer feces-transplanted F344 rats (Fig. 9C). From these results, it was confirmed that, in PH model rats, by reconstituting a gut microbiota of PH patients, the PH pathology becomes more likely to appear.

When RNA-seq analysis of lungs of PH patient feces-transplanted F344 rats and healthy volunteer feces-transplanted F344 rats was performed, in the lungs of the PH patient feces-transplanted F344 rats, RNA expression of Egr1, Cma1, Mcp1, Tnf, Gzmb, and the like was enhanced (Fig. 10A). Further, from results of Gene Ontology term analysis, in PH patient feces-transplanted F344 rats, as compared with healthy volunteer feces-transplanted F344 rats, gene groups related to a defense response against Gram-positive bacteria, gene groups that promote an inflammatory response, and gene groups related to a response to xenobiotic stimulus were enhanced (Fig. 10B), and in particular, genes related to macrophage chemotaxis (C5ar1, Cxcl17, and the like), lymphocyte chemotaxis (Ccl9, Ccl6, and the like), protein processing (Cma1, Gzmb, and the like), an IL-6 response (Mcpt1, Prnp, and the like), and xenobiotic stimulation (Cebpa, Egr1, and the like) were found to be upregulated (Fig. 10C). On the other hand, in healthy volunteer feces-transplanted F344 rats, gene groups related to angiogenesis and vascular formation were enhanced (Fig. 10B), and in particular, genes related to angiogenesis (Tmem100, Sox17, and the like) were upregulated (Fig. 10C).

### 3-6. In PH Model Rats, Promotion of PH Pathology Due to Reconstitution of Gut Microbiota of PH Patients Can Be Canceled by Reducing Gram-Positive Cocci with Vancomycin.

The effect of administration of vancomycin to the PH model gnotobiotic rats on the PH pathology was examined (Fig. 11A). Specifically, a fecal dilution derived from PH patients or healthy volunteers was administered to 11-week-old germ-free F344 rats. From the next day, allowing free drinking of water containing vancomycin (1 g/L) or water was maintained for 4 weeks. Monocrotaline at 60 mg/kg was subcutaneously administered at 15 weeks of age, and after housing the rats for 3 weeks under the normoxic environment (normoxia), the right ventricular systolic pressure and the Fulton's index were measured.

As a result, in a group administered vancomycin, the right ventricular systolic pressure and the Fulton's index were significantly decreased as compared with a group administered water (Fig. 11B). That is, vancomycin-sensitive bacteria (mainly Gram-positive cocci) are involved in the PH pathology, and it has been revealed that, by reducing Gram-positive cocci with vancomycin, promotion of the PH pathology due to reconstitution of the gut microbiota of PH patients can be canceled by reducing Gram-positive cocci with vancomycin.

### 4. Test Results

### 4-1. Gut Microbiota of PH Patients Has Enhanced Tryptophan Metabolism, and Abundant AHR Ligands Are Produced

A mechanism by which dysbiosis of a gut microbiota characteristic of PH patients causes exacerbation of the PH pathology was explored. First, whole genome shotgun sequencing was performed using fecal samples of 52 healthy volunteers and 71 patients. Using the KEGG module database, a metagenomic biological pathway analysis that was significantly changed in PH patients as compared with healthy volunteers was performed. As a result, it was found that, among the pathway modules significantly enhanced in PH patients, the fifth enhanced pathway was tryptophan metabolism (Fig. 12A), and the tryptophan metabolism of the gut microbiota of PH patients was enhanced as compared with the gut microbiota of healthy volunteers. In addition, the AHR ligand activity in fecal samples of PH patients and healthy volunteers was measured by the AHR luciferase reporter assay. As a result, AHR ligand activity was significantly increased in the fecal samples of PH patients as compared with the fecal samples of healthy volunteers (Fig. 12B). That is, these results revealed that abundant AHR ligands are produced using tryptophan as a raw material in the intestine of PH patients.

In addition, the AHR luciferase reporter assay was performed on feces of the PH model gnotobiotic rats prepared above (PH patient feces-transplanted F344 rats and healthy volunteer feces-transplanted F344 rats). As a result, it was confirmed that the AHR ligand activity in feces of the PH patient feces-transplanted F344 rats was significantly increased as compared with that of the healthy volunteer feces-transplanted F344 rats (Fig. 12C), and the characteristics of the gut microbiota of the PH patients were also reflected in the PH patient feces-transplanted F344 rats.

Next, in order to identify AHR ligands derived from the gut bacteria that are key to the pathogenesis, target LC-MS analysis was performed on fecal samples of PH patients and healthy volunteers to determine the concentrations of tryptophan and metabolites thereof. As a result, it was found that the concentration of tryptophan metabolites in fecal samples was increased in the PH patients as compared with that of the healthy volunteers (Fig. 13A). In particular, the concentrations of ILA, Trp, TryptA, IPyA, IAA, and IAld in fecal samples were significantly higher in PH patients than in healthy volunteers, and the upward trend was also observed for IAcr, IPA, and IEt.

In addition, the concentrations of tryptophan and metabolites thereof in feces of the PH model gnotobiotic rats (PH patient feces-transplanted F344 rats and healthy volunteer feces-transplanted F344 rats) prepared above were determined by target LC-MS analysis. As a result, it was found that the concentration of TryptA in feces of the PH patient feces-transplanted F344 rats was significantly increased as compared with that of the healthy volunteer feces-transplanted F344 rats (Fig. 13B).

From these results, it was found that the gut microbiota of PAH patients has the ability to produce indole compounds centered on TryptA, and is involved in the exacerbation of PH pathology caused by dysbiosis of a gut microbiota. Further, in feces of PH patients, the tryptophan concentrations are high, and the concentration of AHR ligands (indole compounds) produced as metabolites of tryptophan is also increased, and it was confirmed that the concentration of tryptophan and metabolites thereof in feces or intestinal contents, and AHR activity in feces or intestinal contents, can be used as examination markers for PH.

### 4-2. Exacerbation of Pathology by Gut Microbiota of PH Patient Is Canceled by AHR Inhibitor

The effect of administration of an AHR inhibitor to the PH model gnotobiotic rats on the PH pathology was examined (Fig. 14A). Specifically, a fecal dilution derived from PH patients was administered to 11-week-old germ-free F344 rats, and monocrotaline was subcutaneously administered at the time of 15 weeks of age. From the day after monocrotaline administration, 8 mg/kg/day of CH223191, which is an AHR inhibitor, or corn oil was administered by daily gavage. After 21 days from the monocrotaline administration, the right ventricular systolic pressure and the Fulton's index were measured, and immune cells were further collected from the lungs. In the group administered the AHR inhibitor (CH223191), both right ventricular systolic pressure and the Fulton's index were significantly improved as compared with the group administered corn oil (vehicle) (Fig. 14B).

Therefore, single-cell RNA-seq analysis was performed to evaluate the effect of CH223191 on the AHR signal-dependent immune cell change in the lungs. Specifically, based on the results obtained by performing single-cell RNA-seq analysis of immune cells collected from the lungs, Uniform Manifold Approximation and Projection (UMAP) was generated to visualize the characteristics of gene expression patterns. As a result, it was found that in the group administered the AHR inhibitor (CH223191), the proportion of Ifny positive cells was significantly decreased in Cluster3 (Th17 cells) expressing IL17a, and Th17 cells were changed to pathogenic Th17 cells in an AHR-dependent manner (Fig. 14C).

Taken together, these results suggest that Th17 cells in the lungs are key to promotion of the PH pathology. It appears that AHR signaling in the intestinal tract induces the differentiation of effector memory T cells in the MLN and the subsequent polarization of pathogenic Th17 cells in the lungs, thereby exacerbating PH.

### 4-3. In Intestine of PH Patients, Impairment of Tryptophan Absorption Occurs, Resulting in Increase in Tryptophan Concentrations in Intestinal Contents and Decrease in Tryptophan Concentrations in Serum

A multicomponent simultaneous LC-MS analysis was performed on fecal samples and serum samples of healthy volunteers and PH patients. As a result, in PH patients, the tryptophan concentrations in the fecal samples were high, whereas the tryptophan concentrations in the serum samples were low (Fig. 15).

In addition, the tryptophan concentrations were measured for fecal samples and serum samples of healthy volunteers, PH patients, and heart failure patients. Regarding the tryptophan concentrations in fecal samples, PH patients showed a significant increase as compared with healthy volunteers, whereas regarding the tryptophan concentrations in serum samples, PH patients showed a significant decrease as compared with healthy volunteers and heart failure patients (Figs. 16A and 16B). In addition, when the tryptophan concentrations were measured for serum samples collected at fasting from healthy volunteers and PH patients, the tryptophan concentrations in PH patients were significantly lower than those in healthy volunteers (Fig. 16C). When disease specificity of this phenomenon was examined, it was shown to be specific to PH patients and was not observed in heart failure patients (Figs. 16A and 16B).

From these results, it was suggested that, in PH patients, impairment of tryptophan absorption occurs, resulting in an increase in tryptophan concentrations in feces and intestinal contents and a decrease in tryptophan concentration in blood.

### 4-4. In Intestine of PH Patients, Neutral Amino Acid Transport Activity Is Decreased

Tryptophan is mainly absorbed by B0AT1, which is a neutral amino acid transporter present at a brush border of the small intestine, and it has been reported that ACE2 regulates expression and function thereof as a chaperone of B0AT1. Here, amino acid concentrations in serum samples of healthy volunteers and PH patients were measured by LC-MS. As a result, in serum samples of PH patients, concentrations of tryptophan, valine, proline, asparagine, threonine, alanine, and methionine, which are neutral amino acids, were significantly decreased, whereas a decrease in concentrations of an acidic amino acid (glutamic acid) and a basic amino acid (lysine) was not observed (Fig. 17). From these results, it was found that neutral amino acid transport activity was reduced in the intestine of PH patients.

### 4-5. In Intestinal Epithelial Cells, Expression of ACE2 Protein Is Decreased in AHR-Dependent Manner

In order to verify the effect of tryptamine (TryptA) having high concentration in the intestine of PH patients on intestinal cells, an in vitro experiment using CACO-2 cells, which are an intestinal epithelial cell line, was performed.

Specifically, after culturing for 72 hours by adding tryptamine to a medium containing CACO-2 cells to be 0 to 500 µM, cells were collected, and the expression level of ACE2 protein was measured. As a result, a decrease in expression level of ACE2 protein in CACO-2 cells was observed depending on the concentration of tryptamine (Fig. 18A).

Specifically, after culturing for 72 hours by adding 200 µM of tryptamine and 0 to 5 µM of AHR inhibitor to a medium containing CACO-2 cells, cells were collected, and the expression level of ACE2 protein was measured. As a result, it was found that the decrease in the expression level of ACE2 protein caused by tryptamine was inhibited by the AHR inhibitor (BAY-218) (Fig. 18B). That is, these results revealed that the expression of ACE2 protein was reduced in intestinal epithelial cells in an AHR-dependent manner.

### 4-6. Tryptamine Decreases B0AT1 (Slc6a19) Function via Decrease in ACE2 Expression in AHR-Dependent Manner and Inhibits Absorption of Tryptophan.

After orally administering tryptamine at 100 mg/kg/day by mixing in a diet for one week to wild-type SD rats and Ahr knockout (Ahr-KO) rats, the small intestine was excised, and the Western blotting analysis and the ex vivo uptake experiment of tritium-labeled tryptophan were performed (Fig. 19A). In the wild-type SD rats, the expression level of the ACE2 protein was decreased by tryptamine administration, whereas in the Ahr-KO rats, the expression level of the ACE2 protein was not decreased by tryptamine administration (Figs. 19B and 19C). Further, as a result of the ex vivo tryptophan uptake experiment, in the wild-type SD rats administered tryptamine, the uptake amount of tryptophan in the small intestine was decreased, whereas in the Ahr-KO rats administered tryptamine, a decrease in uptake amount of tryptophan in the small intestine was not observed (Fig. 19F).

Furthermore, small intestines of wild-type SD rats, Slc6a19 knockout (Slc6a19-KO) rats, and Ace2 knockout (Ace2-KO) rats were excised, and tritium-labeled tryptophan uptake experiments were performed. In the Slc6a19-KO rats in which Slc6a19 (B0AT1), a neutral amino acid transporter, was knocked out, the sodium-dependent tryptophan uptake was reduced (Fig. 19D) along with the decrease in the allele number of Slc6a19 (wild type (+/+) → hetero (+/-) → homo (-/-)). Further, also in the Ace2-KO rats, the sodium-dependent tryptophan uptake was decreased (Fig. 19E).

### 4-7. High Tryptophan Diet Loading Promotes PH Pathology via AHR Agonist Increase

The effect of high tryptophan diet loading in the PH model gnotobiotic rats on the PH pathology was examined (Fig. 20A). Specifically, 11-week-old germ-free F344 rats were administered a fecal dilution derived from PH patients. From 13 weeks of age, the rats were fed either a low tryptophan-containing diet (0.1 w/w% tryptophan) or a high tryptophan-containing diet (1.25 w/w% tryptophan). Monocrotaline was subcutaneously administered at 15 weeks of age, and 21 days after monocrotaline administration, feces and serum were collected, and the right ventricular systolic pressure and the Fulton's index were measured.

When the AHR activity in feces and serum was measured by the AHR luciferase reporter assay, the AHR activity in feces and serum was increased in the group fed the high tryptophan-containing diet as compared with the group fed the low tryptophan-containing diet (Fig. 20B). Furthermore, in the group fed the high tryptophan-containing diet, the right ventricular systolic pressure and the Fulton's index increased, and the PH pathology was exacerbated (Fig. 20C). In addition, when the tryptophan metabolite (indole compound) concentrations in feces were measured by LC-MS, an increase in tryptophan metabolite (indole compound) concentrations was observed in the group fed the high tryptophan-containing diet (Fig. 20D). That is, from these results, it could be confirmed that tryptophan in the intestinal tract is important for the pathogenesis of PH.

### 4-8. AHR Ligand Inhibits Uptake of Tryptophan via Decrease in Expression of ACE2 in Intestinal Epithelial Cells, and Positive Feedback Loop Exists That Enriches AHR Ligands in Intestine

From the results shown above, it is considered that the PH pathology is formed based on the following mechanisms (1) to (4).
(1) Dysbiosis of the gut microbiota occurs by colonization of oral bacteria that produce tryptophan metabolites (indole compounds), which are AHR ligands, in the gut microbiota.
(2) In response to dysbiosis of the gut microbiota, production of AHR ligands (tryptophan metabolites) from tryptophan in the intestinal tract is enhanced.
(3) The accelerated production of AHR ligands (tryptophan metabolites) promotes the differentiation of Th17 cells, which then migrate to the lungs and forms the PH pathology.
(4) The accelerated production of AHR ligands (tryptophan metabolites) in the intestinal tract leads to the AHR-dependent decrease in ACE2 expression to result in impairment of tryptophan absorption. This further increases the tryptophan concentrations in the intestinal tract are further and the vicious cycle is generated in which production of AHR ligands (tryptophan metabolites) in the intestinal tract is further enhanced.

### 4-9. Tryptamine, Which Is AHR Ligand, Exacerbates PH Pathology

The effect of tryptamine administration to germ-free rats on the PH pathology was examined (Fig. 21A). Specifically, 11-week-old germ-free F344 rats were fed either a standard diet or a diet containing 1.25 w/w% tryptamine, and at 15 weeks of age, monocrotaline was subcutaneously administered. At 21 days after monocrotaline administration, the right ventricular systolic pressure and the Fulton's index were measured, and further, the lungs were collected for histological analysis of the pulmonary vascular tissue.

In the group fed the tryptamine-containing diet, the right ventricular systolic pressure and the Fulton's index were significantly increased as compared with the group fed the standard diet (Fig. 21B). In addition, from the Elastica van Gieson (EVG) staining image of the lungs, in the rats fed the diet containing 1.25 w/w% tryptamine, the pulmonary small artery showed medial thickening, and infiltration of inflammatory cells was observed (Fig. 21C).

### 4-10. Ruminococcus Gnavus Exacerbates PH by Cooperative Action with Streptococcus Pasteurianus

In order to identify a TryptA-producing bacterium, metabolic enzymes were focused on, and examination was performed from metagenomic information. TryptA is produced from tryptophan by an L-tryptophan decarboxylase (K01593). Therefore, whole genome analysis (shotgun analysis) was performed using fecal samples of healthy volunteers and PH patients, and a prevalence of a gene (K01593) encoding tryptophan decarboxylase was determined. As a result, it was found that the proportion of bacteria carrying the K01593 gene in PH patients was significantly higher than that in healthy volunteers (Fig. 22A). Further, when species proportion of bacteria carrying the K01593 gene was analyzed, as bacteria carrying the K01593 gene, there were Ruminococcus gnavus, Streptomyces ficellus, Faecalicatena sp., Marseille, Cupriavidus metalligurans, Bradyrhizobium, Sphingopyxis macrogoltabida, and Blautia hansenii, and a majority thereof was accounted for by the R. gnavus (Fig. 22B).

In addition, R. gnavus, S. salivarius, S. Pasturianus, and S. mutans were anaerobically cultured in vitro, and tryptamine in the culture medium was measured over time. As a result, it was confirmed that among these bacteria, only R. gnavus produced tryptamine (Fig. 22C).

From the above results, it was considered that R. gnavus is a pathogen that produces TryptA and exacerbates PH. Therefore, next, the administration experiment of R. gnavus and S. pasteurianus to germ-free rats was performed (Fig. 22D). Specifically, 11-week-old germ-free F344 rats were inoculated with each of R. gnavus and S. pasteurianus alone or in combination, and these bacteria were allowed to colonize the intestine over 4 weeks. At 15 weeks of age, monocrotaline was subcutaneously administered, and 21 days after the monocrotaline administration, the right ventricular systolic pressure and the Fulton's index were measured, qPCR analysis of small intestinal epithelial cells was performed, and the concentrations of tryptophan and the metabolites thereof in feces were measured.

No significant increase in right ventricular systolic pressure and Fulton's index was observed when inoculated with R. gnavus or S. pasteurianus. However, an additive and significant increase in both right ventricular systolic pressure and Fulton's index was observed when these bacteria were inoculated in a combination of R. gnavus and S. pasteurianus (Fig. 22E). Since R. gnavus coexisted with oral bacteria such as Streptococcus and correlated with PH severity, it was considered that Streptococcus may support the growth of R. gnavus.

Streptococcus is known to act on small intestinal epithelial cells of a host to promote mucin production. In fact, from the results of qPCR of small intestinal epithelial cells, it was also confirmed that production of mucin in small intestine epithelial cells was increased only in the presence of S. pasteurianus (Fig. 22F). In addition, in order to confirm the effect of mucin on the proliferation of R. gnavus, a culture experiment of R. gnavus was performed in the presence or absence of mucin. As a result, the turbidity was not increased and the proliferation was not promoted even when R. gnavus was anaerobically cultured in vitro in the absence of mucin, but when R. gnavus was anaerobically cultured in vitro in the presence of mucin, the turbidity was increased and the proliferation was promoted (Fig. 22G). That is, from these results, it was shown that R. gnavus produces TryptA, which is important for the formation of PH pathology; meanwhile, Streptococcus promotes the production of mucin of the host, and R. gnavus acts synergistically by utilizing the production of mucin as a carbon source to support proliferation.

Further, when concentrations of tryptophan and metabolites thereof in feces were quantified by LC-MS, in a case of intake of R. gnavus alone, increases in concentrations of tryptamine and IPA were observed (Fig. 22H). Further, when R. gnavus and S. pasteurianus were inoculated in combination, increases in concentrations of ILA, IPA, and the like were also observed (Fig. 22H), suggesting that an increase in AHR activity in feces had occurred.

### 4-11. Adsorbent Carbon Adsorbs and Removes Various Tryptophan Metabolites

The carbonaceous adsorbent AST-120 is an oral adsorbent that adsorbs indoxyl sulfate to inhibit absorption in the intestinal tract, and is widely used as a treatment for chronic renal failure patients. Therefore, it was confirmed whether tryptophan metabolites could be removed from the intestine by administration of AST-120. Specifically, a standard diet or a diet containing 8 w/w% AST-120 was fed to SD rats at 6 weeks of age for 1 week, and thereafter tryptophan metabolites in feces were analyzed using LC-MS (Fig. 23A). As a result, in the group administered AST-120, a marked decrease in tryptophan metabolites in feces (TryptA, IAld, IAA, IEt, IPA, and ILA) was observed (Figs. 23B to 23G). On the other hand, even when AST-120 was administered, a decrease in tryptophan in feces was not observed (Fig. 23H). From these results, it was confirmed that the administration of the carbonaceous adsorbent can remove tryptophan metabolites (indole compounds) without reducing tryptophan, which is an essential amino acid, in the intestine.

### 4-12. Adsorbent Carbon Adsorbs Tryptophan Metabolites Derived from Gut Bacteria to Improve PH

The effect of administration of the carbonaceous adsorbent AST-120 to the PH model gnotobiotic rats on the PH pathology was examined (Fig. 24A). Specifically, a fecal dilution derived from PH patients was administered to 11-week-old germ-free F344 rats, and monocrotaline was subcutaneously administered at the time of 15 weeks of age. From the day after the monocrotaline administration, a standard diet or a diet containing 8 w/w% AST-120 was fed for 3 weeks, and thereafter measurement of the right ventricular systolic pressure and the Fulton's index, measurement of the AHR activity in feces, and LC-MS analysis of feces were performed.

In a group administered AST-120, the right ventricular systolic pressure and the Fulton's index were significantly suppressed, and the PH pathology was improved (Fig. 24B). In addition, in the group administered AST-120, the AHR activity in feces was also significantly reduced (Fig. 24C). Further, as a result of measuring concentrations of tryptophan and metabolites thereof by LC-MS analysis of feces, in the group administered AST-120, concentrations of tryptophan metabolites (TryptA, IAld, IAA, IEt, IPA, and ILA) in feces were significantly decreased, whereas no decrease in tryptophan concentrations in feces was observed (Fig. 24D). That is, from these results, it was confirmed that the PH pathology can be improved by removing the tryptophan metabolites in the intestinal tract by the carbonaceous adsorbent.

### 4-13. When Regnase-1 CD11C-Cre (Reg-1 CKO) Mice, Severe PAH Spontaneous Onset Model, Are Housed in Germ-Free Environment, Spontaneous Onset of PAH Is Suppressed, and When Adsorbent Carbon Is Administered To Reg-1 CKO Mice, Pathogenesis of PAH Is Suppressed

Regnase-1 CD11c-Cre (Reg-1 CKO) mice, which have been reported as a novel model that spontaneously develops severe PAH, were housed in the SPF environment or the germ-free environment until 12 weeks of age, and then the right ventricular systolic pressure and the Fulton's index were measured. As a result, in Reg-1 CKO mice, the right ventricular systolic pressure was significantly increased under housing in the SPF environment, whereas under housing in the germ-free environment, the increase in right ventricular systolic pressure was suppressed (Fig. 25A). The Fulton's index showed no significant difference in any group (Fig. 25B).

Next, an experiment was conducted to confirm the effect of administration of the carbonaceous adsorbent AST-120 to Reg-1 CKO mice on the spontaneous onset of PAH (Fig. 25C). Specifically, Reg-1 CKO mice at 0 weeks of age were housed for 4 weeks, and thereafter a standard diet or a diet containing 8 w/w% AST-120 was fed for 4 weeks, and the right ventricular systolic pressure and the Fulton's index were measured. As a result, in the Reg-1 CKO mice to which AST-120 was administered, the right ventricular systolic pressure was significantly reduced, and the pathogenesis of PH was suppressed (Fig. 25D).

From these results, it was also confirmed that the dysbiosis of the gut microbiota is involved in the pathogenesis of PH, and the PH pathology can be improved by removing the tryptophan metabolites from the intestinal tract.

### 4-14. Administration of Adsorbent Carbon To Monocrotaline-Loaded PH Model Rats and Hypoxia-Loaded PH Model Rats Suppresses Pathogenesis of PH

The effect of administration of the carbonaceous adsorbent AST-120 to the monocrotaline-loaded PH model rats on the PH pathology was confirmed. Specifically, monocrotaline was subcutaneously administered to male SD rats at 6 weeks of age. After the rats were fed either a standard diet or a diet containing 8 w/w% AST-120 for 3 weeks, the right ventricular systolic pressure was measured. As a result, in the rats to which AST-120 was administered, the right ventricular systolic pressure was significantly reduced, and the pathogenesis of PH was suppressed (Fig. 26A).

Furthermore, the effect of administration of the carbonaceous adsorbent AST-120 to the hypoxia-loaded PH model rats on the PH pathology was confirmed. Specifically, male SD rats at 6 weeks of age were placed in a chamber maintained under 10% oxygen conditions. After the rats were fed either a standard diet or a diet containing 8 w/w% AST-120 for 4 weeks, the right ventricular systolic pressure and the Fulton's index were measured. As a result, in the rats to which AST-120 was administered, the right ventricular systolic pressure was significantly reduced, and the pathogenesis of PH was suppressed (Fig. 26B).

From these results, it was also confirmed that the PH pathology can be improved by removing the tryptophan metabolites from the intestinal tract.

### 4-15. Adsorbent Carbon Adsorbs Indole Compounds

The adsorption capacities of AST-120 (CAS: 90597-58-3; Kureha Corporation), medicinal carbon (CAS: 16291-96-6; Nichi-Iko Pharmaceutical Co., Ltd.), and granulated activated carbon (CAS: 7440-44-0; U.E.S. Co., Ltd.) for various compounds were evaluated. Specifically, each carbonaceous adsorbent (AST-120, medicinal carbon, and granulated activated carbon) was added to the aqueous solution containing 3-aminoisobutyric acid, L-valine, L-glutamic acid, L-tryptophan, tryptamine, indole-3-aldehyde, or indole-3-acetic acid, and an adsorption rate of the carbonaceous adsorbent for each compound was measured. As a result, it was confirmed that AST-120, medicinal carbon, and granulated activated carbon all exhibited low adsorption rates for 3-aminoisobutyric acid, L-valine, and L-glutamic acid, but exhibited high adsorption rates for L-tryptophan, tryptamine, indole-3-aldehyde, and indole-3-acetic acid (Fig. 27). From this result, it was revealed that the carbonaceous adsorbent can efficiently adsorb indole compounds.

The present disclosure is not limited in any way to descriptions of embodiments and examples of the invention. Various modifications that can be easily conceived by those skilled in the art without departing from the scope of the claims are also included in the present invention. The contents of the documents and the like shown in the present specification are cited by incorporating the entire contents thereof.

## Claims

1. A prophylactic or therapeutic agent for pulmonary hypertension, comprising a substance capable of removing an indole compound that is a tryptophan metabolite from an intestinal tract.

2. The prophylactic or therapeutic agent for pulmonary hypertension according to claim 1, wherein the substance is a substance that adsorbs or binds to an indole compound that is a tryptophan metabolite.

3. The prophylactic or therapeutic agent for pulmonary hypertension according to claim 1 or 2, wherein the indole compound is at least one selected from the group consisting of tryptamine, indole-3-aldehyde, indole-3-pyruvic acid, indole-3-ethanol, indole-3-acetic acid, indole-3-propionic acid, indole-3-acrylic acid, and indole-3-lactic acid.

4. The prophylactic or therapeutic agent for pulmonary hypertension according to claim 1 or 2, wherein the substance is a porous inorganic adsorbent.

5. The prophylactic or therapeutic agent for pulmonary hypertension according to claim 1 or 2, wherein the substance is a carbonaceous adsorbent.

6. A method for treating pulmonary hypertension, comprising a step of removing an indole compound that is a tryptophan metabolite from an intestinal tract of a pulmonary hypertension patient.

7. The method for treating pulmonary hypertension according to claim 6, wherein an indole compound that is a tryptophan metabolite is removed from an intestinal tract of a pulmonary hypertension patient by administering to the pulmonary hypertension patient a therapeutically effective amount of a substance that adsorbs or binds to the indole compound.

8. A method for examining the presence or absence of pulmonary hypertension or the severity of pulmonary hypertension, comprising a step of measuring tryptophan concentrations in feces, intestinal contents, or blood samples collected from a subject.

9. An examination kit for the presence or absence of pulmonary hypertension or the severity of pulmonary hypertension, comprising a reagent for measuring tryptophan.

10. A method for examining the presence or absence of pulmonary hypertension or the severity of pulmonary hypertension, comprising a step of measuring concentrations of an indole compound that is a metabolite of tryptophan, in feces, intestinal contents, or blood samples collected from a subject.

11. The examination method according to claim 10, wherein the indole compound is at least one selected from the group consisting of tryptamine, indole-3-aldehyde, indole-3-pyruvic acid, indole-3-ethanol, indole-3-acetic acid, indole-3-propionic acid, indole-3-acrylic acid, and indole-3-lactic acid.

12. An examination kit for the presence or absence of pulmonary hypertension or the severity of pulmonary hypertension, comprising a reagent for measuring an indole compound that is a tryptophan metabolite.

13. A method for examining the presence or absence of pulmonary hypertension or the severity of pulmonary hypertension, comprising a step of measuring an activity of an aryl hydrocarbon receptor in feces or intestinal contents collected from a subject.

14. An examination kit for the presence or absence of pulmonary hypertension or the severity of pulmonary hypertension, comprising a reagent for measuring an activity of an aryl hydrocarbon receptor in feces or intestinal contents.

15. A method for examining the presence or absence of pulmonary hypertension or the severity of pulmonary hypertension, comprising a step of measuring angiotensin-converting enzyme 2 concentrations in feces, intestinal contents, or blood samples collected from a subject.

16. An examination kit for the presence or absence of pulmonary hypertension or the severity of pulmonary hypertension, comprising a reagent for measuring angiotensin-converting enzyme 2.

17. A prophylactic or therapeutic agent for pulmonary hypertension, comprising a substance that increases an expression level of angiotensin-converting enzyme 2.

18. A method for treating pulmonary hypertension, comprising a step of administering a therapeutically effective amount of a substance that increases an expression level of angiotensin-converting enzyme 2, to a pulmonary hypertension patient.
